# EUROPEAN PATENT APPLICATION

(11) **EP 4 576 109 A1**
(43) Date of publication of application: **25.06.2025**
(21) Application number: 24219303.5
(22) Date of filing: 12.12.2024
(51) Int. Cl.: G16H 20/17, G16H 40/63, G16H 70/40, A61M 5/172

(54) **INTELLIGENTLY CONTROLLING PATIENT-CONTROLLED DRUG DELIVERY**

(30) Priority: 20.12.2023 US 202363612544 P
(71) Applicant: CareFusion 303, Inc., San Diego, CA 92130 (US)
(72) Inventor: Abal, Daniel M., San Diego, 92130 (US); Burgess, Brendan John, San Diego, 92130 (US)
(74) Representative: Zenz Patentanwälte Partnerschaft mbB

(57) **Abstract**

An infusion control device detects a patient-controlled drug-requesting device that is in operable communication with the infusion control device. The infusion control device identifies sensor devices and drug-delivery apparatuses separate from the infusion control device that are in operable communication with the infusion control device. The infusion control device selects a drug-control algorithm for approving drug requests received by the patient-controlled drug-requesting device. The infusion control device identifies a patient using the patient-controlled drug-requesting device, and receives patient physiological data from the sensor devices. The infusion control device receives a request for a drug to be delivered to the patient by a drug-delivery apparatus. The infusion control devices determines whether the patient is authorized to perform the drug request. And based on determining that the patient is authorized, the infusion control device causes delivery of the drug to the patient.

## Description

### TECHNICAL FIELD

This application relates generally to control of infusion devices.

### BACKGROUND

Patient-controlled analgesia (PCA) is a method used for clinical pain relief that can be more convenient for both the clinician and the patient. The method typically involves using a syringe pump programmed to allow for delivery of a predefined amount of analgesia (e.g., a bolus dose) when the patient desires it. Some implementations of PCA include a device for the patient to request delivery of the analgesia. There may be instances when a patient should not receive a dose of a drug (e.g., analgesia). For example, the patient may be unconscious or otherwise in an unstable condition, over-sedated, or someone may be diverting the drug away from the patient. However, safety features for stopping administration of medication are limited and may only be designed for particular devices implementing such features.

### SUMMARY

Thus, there is a growing need for PCA control devices that implement drug-control algorithms to control drug delivery, such as by conventional syringe pumps. The present disclosure discusses various implementations of devices, systems, and methods for implementing a pause control (e.g., via a drug-control algorithm) at various drug-delivery apparatuses (e.g., conventional syringe pumps, fluid infusion pumps) by determining which devices (e.g., sensor devices) are in the systems containing the drug-delivery apparatuses, and the identity of the patients using the systems.

According to various implementations, systems include a patient-controlled drug-requesting device and one or more drug-delivery apparatuses that are in operable communication with the patient-controlled drug-requesting device. An infusion control device can interchangeably couple to any of the various patient care systems and implement pause control of drugs that a patient of the system is able to request. For example, a clinician may couple the infusion control device to a patient care system that includes a conventional syringe pump, and a patient-controlled drug-requesting device with an input that the patient can use to request doses from the syringe. The infusion control device can implement a drug-control algorithm to control the patient's use of the patient-controlled drug-requesting device by authorizing requests for drug delivery by the patient. The devices, systems, and methods described herein include various technical improvements over conventional PCA systems. Although the present disclosure will explicitly recite some technical improvements, it will also make other improvements apparent to those of skill in the art by virtue of the disclosed implementations. For example, implementations described herein provide interoperability to PCA systems by implementing pause control at systems that include appropriate sensors, data, and components for controlling delivery of a drug, even when the system does not include any particular software for implementing the control of delivery of the drug. Further, implementations of systems, devices, and methods described herein can improve the functioning of computers and/or computing devices being used in various implementations of PCA systems by optimizing the computing and power requirements of a system that requires pause control for more than one drug and/or when there is more than one way to implement pause control for a particular drug. For example, certain implementations may determine that one or more sensor devices that are in operable communication with a patient care system can provide data for use by drug-control algorithms to control delivery of more than one drug and can reduce power to sensor devices that do not need to be used based on the drugs that the patients can request and/or the drug-control algorithms selected. Further, systems can use the implementations described to control drug delivery in conjunction with other devices (e.g., lockboxes, multi-parameter monitors) that may be separately restricting or otherwise controlling patient access to aspects of the patient care system.

Various implementations of this disclosure use an infusion control device that includes one or more processors and a memory storing instructions, which, when executed by the one or more processors, causes the one or more processors to perform operations comprising the following: detecting a patient-controlled drug-requesting device that is in operable communication with the infusion control device; identifying, based on detecting the patient-controlled drug-requesting device, (i) one or more sensor devices and (ii) one or more drug-delivery apparatuses separate from the infusion control device, wherein the one or more sensor devices and the one or more drug-delivery apparatuses are in operable communication with the infusion control device; selecting, based on identifying the one or more sensor devices and the one or more drug-delivery apparatuses, a drug-control algorithm for approving drug requests received by the patient-controlled drug-requesting device; identifying a patient using the patient-controlled drug-requesting device; receiving patient physiological data from the one or more sensor devices in operable communication with the infusion control device; receiving a request for a drug to be delivered to the patient by a drug-delivery apparatus of the one or more drug-delivery apparatuses; determining whether the patient is authorized to perform the drug request based on a function of the drug-control algorithm, the patient physiological data, and a history of drug administrations to the patient; and causing, based on determination by means of the drug-control algorithm that the patient is authorized to perform the drug request, delivery of the drug via the one or more drug-delivery apparatuses.

According to various aspects, a machine-implemented method comprises, at an infusion control device that includes one or more processors and a memory storing instructions: detecting a patient-controlled drug-requesting device that is in operable communication with the infusion control device; identifying, based on detecting the patient-controlled drug-requesting device, (i) one or more sensor devices and (ii) one or more drug-delivery apparatuses separate from the infusion control device, wherein the one or more sensor devices and the one or more drug-delivery apparatuses are in operable communication with the infusion control device; selecting, based on identifying the one or more sensor devices and the one or more drug-delivery apparatuses, a drug-control algorithm for approving drug requests received by the patient-controlled drug-requesting device; identifying a patient using the patient-controlled drug-requesting device; receiving patient physiological data from the one or more sensor devices in operable communication with the infusion control device; receiving a request for a drug to be delivered to the patient by a drug-delivery apparatus of the one or more drug-delivery apparatuses; determining whether the patient is authorized to perform the drug request based on a function of the drug-control algorithm, the patient physiological data, and a history of drug administrations to the patient; causing, based on the determining by means of the drug-control algorithm that the patient is authorized to perform the drug request, delivery of the drug via the one or more drug-delivery apparatuses.

According to various aspects, a system comprises a drug-delivery device in operable communication with a control unit for controlling drug delivery to a patient via the drug-delivery device, wherein the control unit includes a handheld electronic drug-requesting device, one or more processors, and a memory, including instructions, which, when executed by the one or more processors, cause performance of operations, and comprising the following: determining that a handheld electronic drug-requesting device is electronically coupled to the drug-delivery device for delivery of a drug to the patient; receiving, in response to determining that the handheld electronic drug-requesting device is electronically coupled to the drug-delivery device, information related to a patient profile associated with the patient from the drug-delivery device; presenting, on a display integrated in the handheld electronic drug-requesting device, a user interface element including the information related to the patient profile associated with the patient; receiving, concurrently with the user interface element being presented on the display integrated in the handheld electronic drug-requesting device, a patient request to deliver a dose of the drug to the patient via a touch-activated control of the handheld electronic drug-requesting device; and, responsive to the patient request, causing the dose of the drug to be delivered to the patient. Other aspects include corresponding devices, methods, and computer program products for implementation of the corresponding system and its features.

Other configurations of the subject technology will become readily apparent to those skilled in the art from the following detailed description, wherein various configurations of the subject technology are shown and described by way of illustration. As those skilled in the art will realize, the subject technology is capable of other and different configurations, and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description below, in conjunction with the following drawings. Like reference numerals refer to corresponding parts throughout the figures and description.
FIG. 1 depicts an example of an institutional patient care system of a healthcare organization, according to aspects of the subject technology.
FIG. 2A depicts an example of an institutional patient care system of a healthcare organization, according to aspects of the subject technology.
FIG. 2B is a closer view of a portion of the example patient care device shown in FIG. 2A, according to various aspects of the subject technology.
FIGS. 3A to 3E depict example configurations of a system that includes an infusion control device in operable communication with one or more drug-delivery apparatuses, according to various aspects of the subject technology.
FIG. 4 depicts an example process for implementing a drug-control algorithm at an infusion control device in operable communication with one or more drug-delivery apparatuses, according to various aspects of the subject technology.
FIG. 5 is a conceptual diagram illustrating an example electronic system for operating an analgesia administration system, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

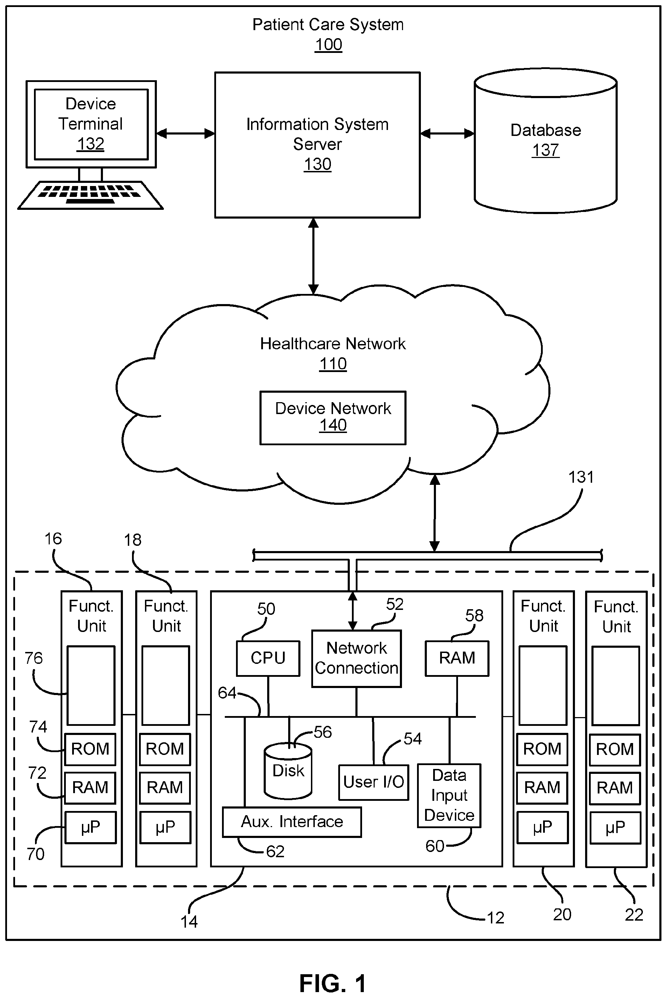

FIG. 1 depicts an example of an institutional patient care system 100 of a healthcare organization, according to aspects of the subject technology. In FIG. 1, a patient care device 12 ("PCD"), sometimes referred to as a "patient care unit" ("PCU") or more generally as a "medical device," is connected to a healthcare network 110. The patient care device 12 may include or be communicatively connected to various ancillary medical devices such as drug-delivery apparatuses (e.g., infusion pumps, syringe pumps), a vital signs monitor, a medication dispensing device (e.g., cabinet, tote), a medication preparation device, an automated dispensing device, a module coupled with one of the aforementioned drug-delivery apparatuses (e.g., a syringe pump module configured to attach to an infusion pump), a patient-controlled analgesia (PCA) wand, or other similar devices. Each element of patient care device 12 may be connected to healthcare network 110 by a transmission channel 131. Transmission channel 131 may be any suitable wired or wireless transmission channel, for example, an 802.11 wireless local area network (LAN). In some implementations, healthcare network 110 also includes computer systems located in various departments throughout a hospital. For example, healthcare network 110 optionally includes computer systems associated with one or more of: an admissions department, a billing department, a biomedical engineering department, a clinical laboratory, a central supply department, one or more unit station computers and/or a medical decision support system. As described further below, healthcare network 110 may include discrete subnetworks. In the depicted example, healthcare network 110 includes a device network 140 by which patient care device 12 (and other devices) may communicate, in accordance with normal operations. According to some implementations, the devices and supporting services of healthcare network 110 or a portion thereof may be cloud-based with, for example, the servers and services (e.g., databased, APIs, etc.) located remote from the hospital and/or distributed across multiple remote locations or regions.

Additionally, institutional patient care system 100 may incorporate a separate information system server 130, the function of which will be described in greater detail below. Moreover, although information system server 130 is shown as a separate server, the functions and programming of information system server 130 may be incorporated into another computer, such as, for example, a hospital information system server or cloud-based server, if so desired by engineers designing the institution's information system. Institutional patient care system 100 may further include one or multiple device terminals 132 for connecting and communicating with information system server 130. Device terminals 132 may include personal computers, personal data assistant, mobile devices such as laptops, tablet computers, augmented reality devices, or smartphones, configured with software for communications with information system server 130 via healthcare network 110. The information system server 130 may receive and provide information about the patient and/or their treatment such as measurements from patient monitoring devices (not shown), entries for the patient via one of the device terminals 132, or events from the patient care device 12.

Patient care device 12 may include or incorporate pumps, physiological monitors (e.g., heart rate, blood pressure, electrocardiogram (ECG), electroencephalogram (EEG), pulse oximeter, and other patient monitors), therapy devices, and other drug delivery devices may be utilized according to the teachings set forth herein (e.g., infusion control devices that are configured to operably couple with patient care device 12 or another device within patient care system 100). In the depicted example, patient care device 12 comprises a control module, also referred to as interface unit 14, connected to one or more functional modules 16, 18, 20, 22. Interface unit 14 includes a central processing unit (CPU) 50 connected to a memory, for example, memory 58 (e.g., random access memory (RAM)), and one or more interface devices such as user interface device 54 (e.g., a display screen and/or keyboard), a coded data input device 60, a network connection 52, and an auxiliary interface 62 for communicating with additional modules or devices. Interface unit 14 also, although not necessarily, includes a main non-volatile storage unit (e.g., a disk 56), such as a hard disk drive or non-volatile flash memory, for storing software and data and one or more internal buses 64 for interconnecting the aforementioned elements.

In various implementations, user interface device 54 is a touchscreen for displaying information to a user and allowing a user to input information by touching defined areas of the screen. Additionally, or in the alternative, user interface device 54 could include any means for displaying and inputting information, such as a monitor, a printer, a keyboard, softkeys, a mouse, a track ball and/or a light pen. Data input device 60 may be a bar code reader capable of scanning and interpreting data printed in bar coded format. Additionally, or in the alternative, data input device 60 can be any device for entering coded data into a computer, such as a device(s) for reading magnetic strips, radio-frequency identification (RFID) devices whereby digital data encoded in RFID tags or smart labels (defined below) are captured by the data input device 60 via radio waves, PCMCIA smart cards, radio frequency cards, memory sticks, CDs, DVDs, or any other analog or digital storage media. Other examples of data input device 60 include a voice activation or recognition device or a portable personal data assistant (PDA). Depending upon the types of interface devices used, user interface device 54 and data input device 60 may be the same device. Although FIG. 1 shows data input device 60 disposed within interface unit 14, it is recognized that data input device 60 may be integral within pharmacy system 34 or located externally and communicating with pharmacy system 34 through an RS-232 serial interface or any other appropriate communication means. Auxiliary interface 62 may be an RS-232 communications interface, however any other means for communicating with a peripheral device such as a printer, patient monitor, infusion pump or other medical devices may be used without departing from the subject technology. Additionally, data input device 60 may be a separate functional module, such as functional modules 16, 18, 20 and 22, and configured to communicate with interface unit 14, or any other system on the network, using suitable programming and communication protocols. Network connection 52 may be a wired or wireless connection, such as by Ethernet, WiFi, BLUETOOTH, an integrated services digital network (ISDN) connection, a digital subscriber line (DSL) modem or a cable modem. Any direct or indirect network connection may be used, including, but not limited to a telephone modem, an MIB system, an RS232 interface, an auxiliary interface, an optical link, an infrared link, a radio frequency link, a microwave link or a WLANS connection or other wireless connection.

Functional modules 16, 18, 20, 22 are any devices associated with interface unit 14 for providing care to a patient and/or for monitoring the patient's condition. As shown in FIG. 1, at least one of functional modules 16, 18, 20, 22 may be an infusion pump module such as an intravenous infusion pump for delivering medication or other fluid to a patient. For example, functional module 16 may be an infusion pump module. Each of functional modules 16, 18, 20, and 22 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart-rate monitor, or an intracranial pressure monitor or the like. In other examples, functional modules 18, 20 and/or 22 may include devices other than infusion pump modules, including a printer, scanner, bar code reader or any other peripheral input, output, or input/output device.

Each functional module 16, 18, 20, and 22 communicates directly or indirectly with interface unit 14, with interface unit 14 providing overall monitoring and control of patient care device 12. Functional modules 16, 18, 20, and 22 may be connected physically and electronically in serial fashion to one or both ends of interface unit 14 as shown in FIG. 1. However, it is recognized that there are other means for connecting functional modules with the interface unit that may be utilized without departing from the subject technology. It will also be appreciated that devices such as pumps or patient monitoring devices that provide sufficient programmability and connectivity may be capable of operating as stand-alone devices and may communicate directly with the network without requiring a connection through separate interface unit 14. As described above, additional medical devices or peripheral devices may be connected to patient care device 12 through one or more auxiliary interfaces 62.

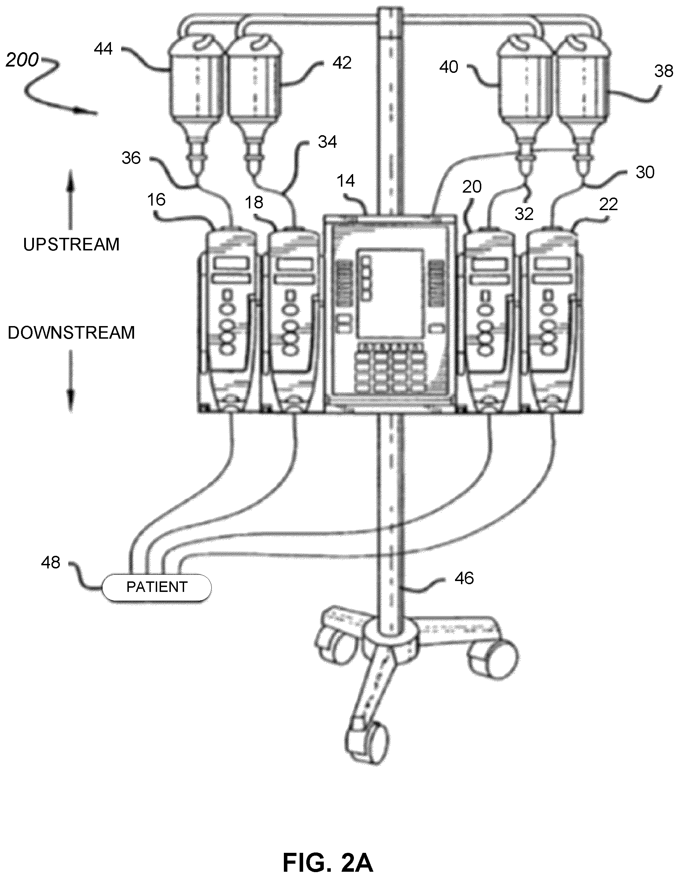

Each of functional modules 16, 18, 20, and 22 may include module-specific components 76, a microprocessor 70, a volatile memory 72 and a nonvolatile memory 74 for storing information. In some implementations, a functional module may include hardware components similar to those of interface unit 14 including, but not limited to, a CPU 50 connected to memory 58, one or more interface devices such as user interface device 54, a coded data input device 60, a network connection 52, and an auxiliary interface 62 for communicating with additional modules or devices. It should be noted that while four functional modules are shown in FIG. 1, any number of devices may be connected directly or indirectly to interface unit 14. The number and type of functional modules described herein are intended to be illustrative, and in no way limit the scope of the subject technology. Module-specific components 76 include any components necessary for operation of a particular module, such as a pumping mechanism for an infusion pump module (e.g., an infusion pump module 22 as shown in FIGS. 2A-2B).

According to various implementations, while each functional module may be capable of independent operation (e.g., as described with respect to interface unit 14 and its hardware components), interface unit 14 is configured to monitor and control overall operation of patient care device 12. For example, interface unit 14 may provide programming instructions to the functional modules 16, 18, 20, 22 and monitor the status of each module.

Medical devices incorporating aspects of the subject technology may be equipped with a Network Interface Module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, patient care device 12 and healthcare network 110 may communicate via automated interaction, manual interaction, or a combination of both automated and manual interaction. Automated interaction may be continuous or intermittent and may occur through network connection 52 (as shown in FIG. 1), or through RS232 links, MIB systems, RF links such as BLUETOOTH, IR links, WLANS, digital cable systems, telephone modems or other wired or wireless communication means. Manual interaction between patient care device 12 and healthcare network 110 involves physically transferring, intermittently or periodically, data between systems using, for example, user interface device 54, coded data input device 60, bar codes, computer disks, portable data assistants, memory cards, or any other media for storing data. The communication means in various aspects is bidirectional with access to data from as many points of the distributed data sources as possible.

FIG. 2A depicts an example of an institutional patient care system of a healthcare organization, according to aspects of the subject technology. Patient care device 200 shown in FIG. 2A is similar or identical to patient care device 12 in FIG. 1, including four fluid infusion pumps 16, 18, 20, and 22 (e.g., which are also referred to as functional modules 16, 18, 20, and 22 in FIG. 1), each of which is in operative engagement with a respective fluid administration set 30, 32, 34, and 36. Fluid supplies 38, 40, 42, and 44, which may take various forms but in this case are shown as bottles, are inverted and suspended above the pumps. Fluid supplies may also take the form of bags or other types of containers. In the depicted example, both patient care device 200 and fluid supplies 38, 40, 42, and 44 are mounted to a roller stand or pole 46. The specific fluid supplies as well as their orientation (e.g., mount location, mount height, mounting type, etc.) within the care area may be generate one or more interaction records. The interaction record for a set for example may be generated in part by detecting a scannable code associated with the set prior to use. Once scanned, the interaction record may be recorded for use as described herein.

As shown in the example implementation of FIG. 2A, each administration set 30, 32, 34, and 36 is connected between a respective fluid supply 38, 40, 42, and 44 and a patient 48 so that patient 48 may receive the fluids in all the fluid supplies. The administration set may be identified either actively by, for example, scanning by a clinician or passively by, for example, wireless or optical detection of the administration set. As with the fluid supply, once identified, an interaction record may be generated identifying the administration set and one or more of the clinician, programming module, pump, administration set positioning (e.g., administration location (e.g., left forearm, right upper-arm, etc.).

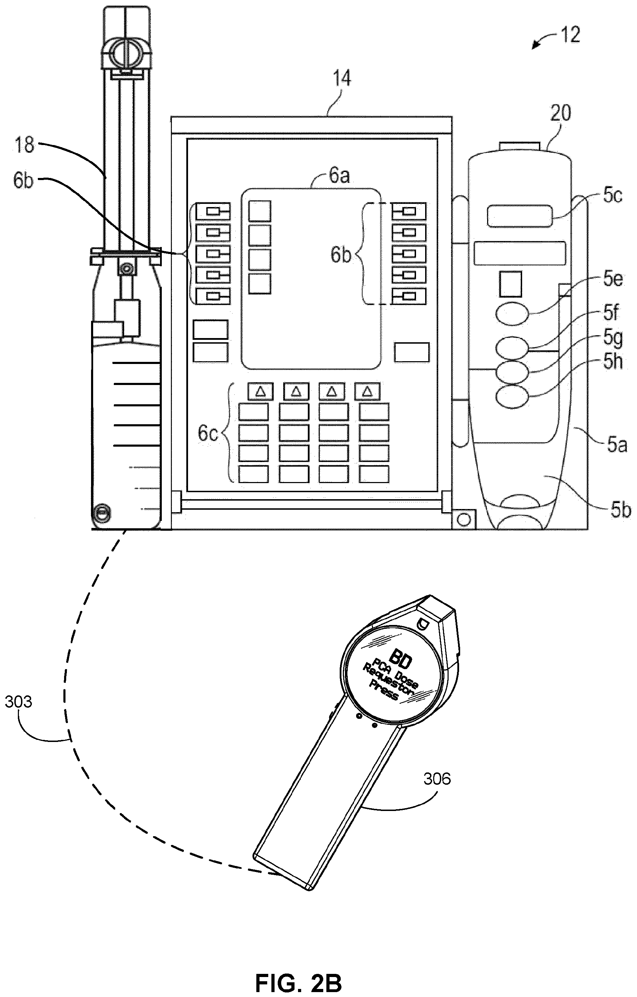

Each of fluid infusion pump 16, 18, 20, and 22 can be used to infuse each of the fluids of the fluid supplies into patient 48. Fluid infusion pumps 22, 24, 26, and 28 are flow-control devices that will act on the respective tube or fluid conduit of the fluid administration set to move the fluid from the fluid supply through the conduit to patient 48. Because individual pumps are used, each can be individually set to the pumping or operating parameters required for infusing the particular medical fluid from the respective fluid supply into the patient at the particular rate prescribed for that fluid by the clinician. The activities performed by the pump or clinician to infuse the particular medical fluid may be associated with one or interaction which may be recorded and processed as described. FIG. 2B is a closer view of a portion of the example patient care device shown in FIG. 1A, according to various aspects of the subject technology. FIG. 2B shows two functional modules; functional syringe pump module 18 and functional infusion pump module 20, mounted at either side of interface unit 14, and the displays and control keys of each. The functional infusion pump module 20 includes a door 5a and a handle 5b that operates to lock the door in a closed position for operation and to unlock and open the door for access to the internal pumping and sensing mechanisms and to load administration sets for the pump. When the door 5a is open, the tube can be connected with the pump module 20. When the door 5a is closed, the tube is brought into operating engagement with the pumping mechanism, the upstream and downstream pressure sensors, and the other equipment of the pump. A display 5c, such as an LED display, is located in plain view on the door in this embodiment and may be used to visually communicate various information relevant to the pump module 20, such as alert indications (e.g., alarm messages). Control keys 5e to 5h may exist for programming and controlling operations of the infusion pump as desired. In some implementations, the control keys may be presented as interactive elements on the display 5c (e.g., touchscreen display). The main frame and/or functional module may also include audio alert equipment in the form of a speaker (not shown).

The interface unit 14 of the patient care device 12 includes a display 6a for visually communicating various information, such as the operating parameters of a connected pump and alert indications and alert messages, and control keys 6b and 6c for selecting and/or setting control parameters and/or options for controlling the patient care device 12 and connected modules. The interface unit 14 may also include a speaker to provide audible alerts. In some implementations, the display 6a may be implemented as a touchscreen display. In such implementations, the control keys 6b may be omitted or reduced in number by providing corresponding interactive elements via a graphical user interface presented via the display 6a. In some implementations, each control key 6b (or 6c) may select a corresponding option displayed in display 6a.

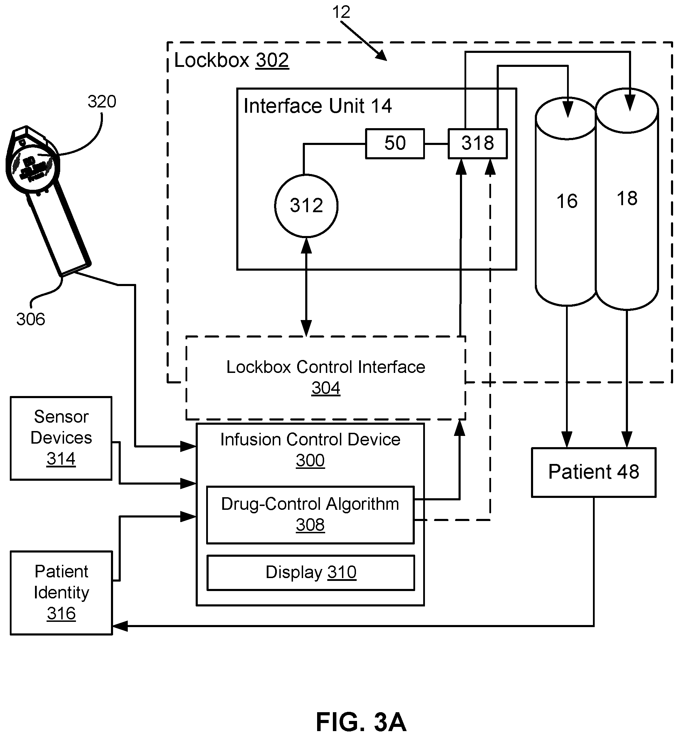

The interface unit 14 may include a communications system (not shown) with which the interface unit 14 may communicate with external equipment such as a medical facility server or other computer and with a portable processor, such as a handheld communication device or a laptop-type of computer, or other information device that a clinician may have to transfer information as well as to download drug libraries to a functional module 16, 18, 20, 22 (e.g., fluid infusion pump 20). In some implementations, the main frame infusion controller may communicate (e.g., through a wired connection or wirelessly) with patient-controlled drug-requesting device 306, which is described in greater detail below with respect to FIG. 3A. The communication system may be used to transfer access and interaction information for clinicians encountering the main frame infusion controller or device coupled therewith (e.g., fluid infusion pump module 22 or bar code scanner). The communications system may include one or more of a radio frequency (RF) system, an optical system such as infrared, a BLUETOOTH^{™} system, or other wired or wireless system. The bar code scanner and communications system may alternatively be included integrally with the infusion pump 20, such as in cases where a main frame infusion controller is not used, or in addition to one with the main frame control unit 14. Further, information input devices need not be hard-wired to medical instruments, information may be transferred through a wireless connection as well. Additionally, other types of modules may be connected to the pump modules or to the main frame infusion controller such as a syringe pump module, patient controlled analgesic module, end tidal CO₂ (ETCO2) monitoring module, oximeter monitoring module, or the like.

For the purpose of this disclosure, interface unit 14 and/or information server 130 may include software and associated algorithms for implementing the various features described herein. This hardware, software, and associated algorithms are collectively referred to herein as the patient care system. In some implementations, interface unit 14 operates the patient care system in that the internal processing and decision control for approval of a dose request made by patient-controlled drug-requesting device 306. In some implementations, the dose request may be forwarded from interface unit 14 to the information server 130 for approval. In some implementations, on the interface unit 14 receiving the dose request, the interface unit may process and approve the dose request based on obtaining patient profile information from the information system server 130 (e.g., from an EMR system).

During operation of the patient care system, when patient 48 activates the connected patient-controlled drug-requesting device 306, interface unit 14 (including, e.g., microprocessor 50) receives a dose request signal via a patient dose request cord 303 (or through a wireless communication). If interface unit 14 determines (e.g., in connection with profile data obtained from an internal database or via information system server 130) that there are no limitations in administering a requested bolus dose of medication, interface unit 14 may then send a signal to a connected pump controller to instruct a pump unit associated with the request to administer the requested bolus dose. The interface unit 14 also provides for the coordination of activities between the functional units, such as the pump and an ETCO2 unit. For example, a clinician may set up the patient care device 12 to provide PCA administration and the ETCO2 unit to monitor the ETCO2 parameters of a PCA patient. Optionally, one or more additional monitors, such as a pulse oximetry unit, may be communicatively connected to the patient care system and set up to monitor blood oxygen saturation and pulse rate. The clinician may specify a minimum and/or maximum value for ETCO2, respiration rate, and/or other monitored parameters which thereby effectively sets a range of acceptable values for those parameters. If the patient's ETCO2 parameter is outside the selected acceptable range, such as in the case where it becomes less than the minimum or greater than the maximum levels set by the clinician, the ETCO2 monitor may send a trigger signal to the interface unit 14. In response, interface unit 14 may activate a visual and/or auditory alarm (e.g., via a display 310 associated with infusion control device 300, as described below), suspend operation of the pump, adjust the flow rate of the pump, and/or perform another predetermined function. For example, in response to an out-of-range ETCO2 measurement in a patient 48, interface unit 14 may cease all further administration of analgesics until after the unacceptably low or high ETCO2 value and/or respiration rate situation is resolved, such as by clinician intervention or patient change. Alternatively, interface unit 14 may simply lock-out the infusion control device 300 so that the patient cannot obtain further self-administrations. Thus, after appropriate values have been set up, the patient control system provides communication and coordination between the interface unit, pump, and the ETCO2 unit ensure greater safety and decreased risk of injuries from respiratory depression.

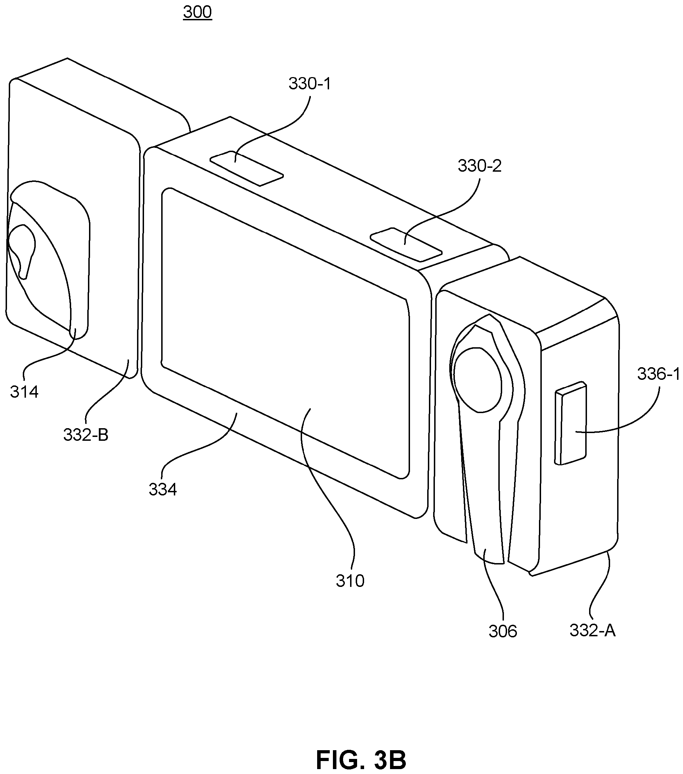

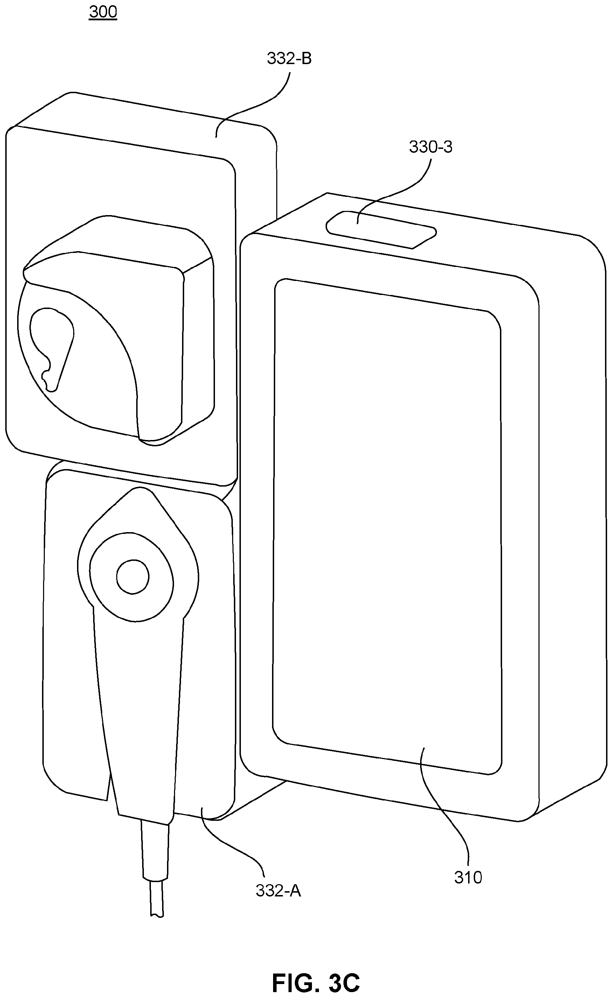

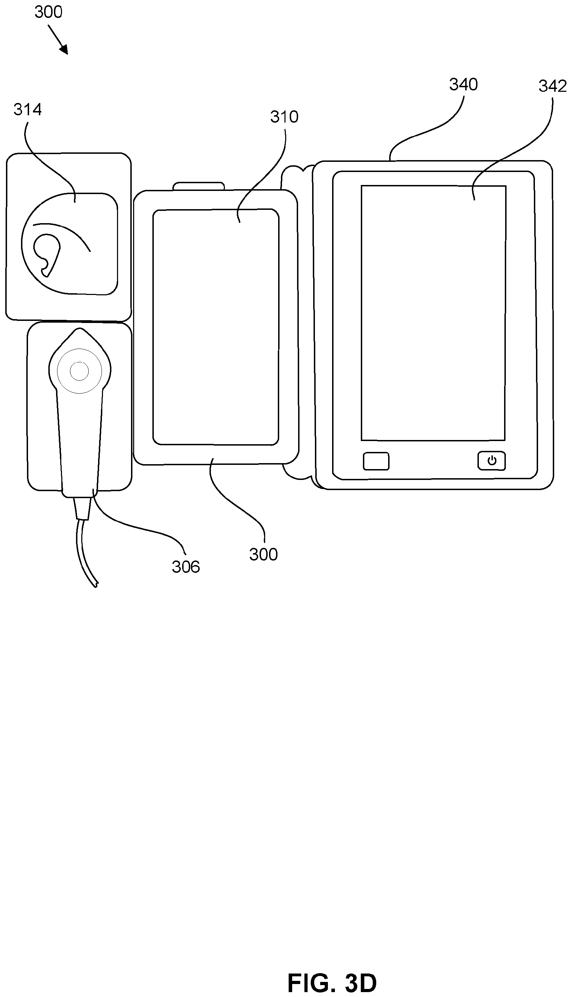

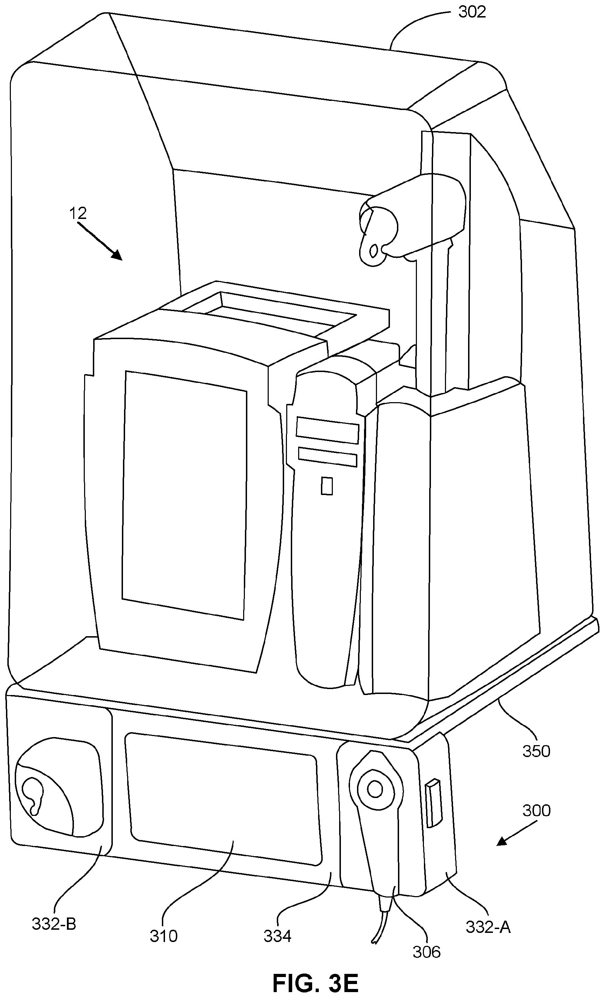

In some implementations, rather than interface unit 14 suspending operation of the pump in response to only an out-of-range signal from the ETCO2 unit or from another functional module, interface unit 14 may include program instructions for monitoring the changes in the CO₂ concentration data or other data generated by the ETCO2 unit and to make decisions on whether to interfere with the patient's control of the pump module based upon the changes, such as the rate of change, in the monitored data. In some implementations, interface unit 14 is configured to receive instructions from an infusion control device (such as infusion control device 300 described with respect to FIG. 3A), including information about decisions on whether to interfere with the patient's control of one or more drug-delivery apparatuses (e.g., pump modules, syringe modules) and/or whether to provide additional instructions for monitoring particular health parameters. Typically, medical fluid administration sets have more parts than are shown in FIGS. 1-2B. Many have check valves, drip chambers, valved ports, connectors, and other devices well known to those skilled in the art. These other devices have not been included in the drawings so as to preserve clarity of illustration. Refer to FIGS. 3-4 and the related description below on how patient-controlled drug-requesting device 306 can be operated in conjunction with components and devices of patient care system 100, including patient care device 200. FIG. 3A to 3E depict example configurations of a system that includes an infusion control device 300 in operable communication with one or more drug-delivery apparatuses (e.g., functional modules 16 and 18, and/or constituent components thereof), according to various aspects of the subject technology. According to various implementations, an infusion control device 300 is provided to interface patient care device 12 with one or more external devices, and to provide control and/or oversite of the patient care device 12 based on those connected external devices. FIG. 3A depicts an example block diagram of the system that includes the infusion control device 300. In some implementations, the infusion control device 300 is a modular device that is separate from the patient care device 12. In this regard, the infusion control device 300 can be detachably coupled to the patient care device 12 as a functional module of the patient care device 12 or additionally or alternatively to any of the functional modules 16, 18, 20, or 22. In the depicted example, the infusion control device 300 forms a communication connection to the interface unit 14 of the patient care device 12 through a communication connection formed with the lockbox 302, configured and arranged for securing a portion of the patient care device 12 (e.g., the interface unit 14 and/or the functional modules 16 and 18). In some implementations, the infusion control device 300 can be detachably coupled to the patient care device 12 without being connected to the lockbox 302. That is, the infusion control device 300 may be interoperable so as to work with different configurations of patient care systems, such as being able to couple either directly to the patient care device 12 or a component housing the patient care device 12 (or a portion thereof), such as the lockbox 302. In some implementations, the infusion control device 300 includes a first type of connector for coupling to the patient care device 12 and a second type of connector for coupling to the lockbox 302. In some implementations, the infusion control device 300 is configured to detect the presence of the lockbox 302 while being operably coupled with the patient care device 12. In some implementations, based on detecting that the lockbox 302 is surrounding the patient care device 12, the infusion control device 300 performs additional operations for prompting a patient 48 to provide a credential and for receiving the credential provided by the patient 48 and providing the credential to the patient care device 12.

The infusion control device 300 may include a display module 310 for displaying various information that is likely to be relevant to the patient 48 or a clinician. For example, the display module 310 may indicate statuses of sensor devices 314 and/or drug-delivery apparatuses of the patient care device 12, and/or information about drug requests by the patient 48. In some implementations, the display module 310 of the infusion control device 300 is configured to display one or more user interface elements corresponding to user interface elements displayed at the user interface device 54 of the patient care device 12. When a clinician is authorized to use the patient care device 12, the coupled infusion control device 300 may aggregate interface input systems (e.g., display 6a and/or 5c and/or controls 6b/6c and/or 5e-h) to the clinician via the display module 310 of the infusion control device 300. Such aggregation may be particularly useful when the patient care device 12 is secured by a lockbox 302.

According to various implementations, the lockbox 302 includes a housing that completely encompasses the patient care device 12, including any attached functional modules and associated medication, and secures the patient care device 12 and its respective functional modules from physical manipulation by a person (e.g., patient 48) who does not have access to the lockbox 302. In some implementations, the medication within a syringe pump of the functional modules is secured by way of being enclosed within the lockbox 302. In some implementations, the lockbox 302 may be extended to house and secure a medication bag for use with different types of pumps (e.g., peristaltic pumps). In some implementations, the lockbox 302 is self-powered and includes a power supply within the lockbox 302 (e.g., inaccessible to a person outside the lockbox 302 when the lockbox 302 is secured). In some implementations, the lockbox 302 has one or more automated doors (or lids), and power for the lockbox doors/lids and/or infusion control device 300 may be provided or supplemented by the power supply 312 of the patient care device 12. According to various implementations, the infusion control device 300 may form an electronic communication connection with a lockbox control interface 304 of lockbox 302 where the lockbox control interface 304 is configured to enclose and prevent access to at least a portion of the patient care device 12. In some implementations, after the lockbox control interface 304 establishes the electronic communication connection, the infusion control device 300 receives a wireless credential at an input interface of the infusion control device 300, and the infusion control device 300 transmits the wireless credential to the patient care device 12 via the electronic communication connection within the lockbox control interface 304. In some implementations, after receiving and transmitting the credential, infusion control device 300 receives, from the patient care device 12 via the electronic communication connection, an indication as to whether the patient 48 is authorized to request doses of the drug to be delivered. In some implementations, in accordance with receiving the indication that the patient 48 is authorized to request doses of the drug to be delivered, the infusion control device 300 uses a selected drug-control algorithm (e.g., drug-control algorithm 308) to determine if a drug request submitted by the patient 48 is an authorized drug request. If the patient 48 is not authorized to request doses of the drug, the lockbox control interface 304 and/or the display module 310 of the infusion control device 300 may prevent delivery of the dose and display an indication that the credential was not verified.

According to some implementations, after the infusion control device 300 forms an operable communication connection with the patient care device 12 (e.g., via the communication connection formed with the lockbox 302), the infusion control device 300 detects the patient-controlled drug-requesting device 306 (e.g., a PCA wand, or a dose requester device). The patient-controlled drug-requesting device 306 may be integrally connected or detachably couplable to the infusion control device 300, or the patient-controlled drug-requesting device 306 may be attached to another device of the patient care system 100 (e.g., through an input of data from the input device 60). In some implementations, the data input device 60 can receive a communication from the connection component (e.g., an auxiliary connector such as patient dose request cord 303) of the patient-controlled drug-requesting device 306. In some implementations, the patient-controlled drug-requesting device 306 includes a wireless communication component (e.g., Bluetooth circuitry) and is configured to form a wireless connection with one or more of the infusion control device 300, lockbox 302, interface unit 14, and/or one or more functional modules of the patient care device 12. In some implementations where the patient-controlled drug-requesting device 306 is integrally attached or detachably coupled to the infusion control device 300, the infusion control device 300 provides information about the patient-controlled drug-requesting device 306 to the patient care device 12, which may be provided in conjunction (e.g., concurrently) with a request for data about patient care device 12.

In some implementations, the patient-controlled drug-requesting device 306 includes the display 320, which may be a touchscreen display. The display 320 may be used to display information related to drug delivery, including patient-interactable user interface elements that the patient 48 can select to cause operations to be performed by the patient-controlled drug-requesting device 306, infusion control device 300 and/or a combination of other components of the patient care system 100. For example, the display 320 may first display a user interface element that the patient 48 can select to cause a dose of a drug to be delivered, and may thereafter (e.g., at some predefined duration after the drug has been delivered) display a pain assessment user interface that the patient 48 can provide a response to indicating a level of pain that the patient 48 is experiencing. In some implementations, an interaction by the patient 48 with the pain assessment user interface can be included in the operations of the drug-control algorithm 308 to determine whether the patient 48 is authorized to receive a dose of the requested drug. For example, if the patient 48 indicates that they are experiencing a high level of pain shortly after receiving a dose of the requested drug, the infusion control device 300 may determine that the drug is ineffective for treating a type of pain being experienced by the patient 48, or that the drug is not actually being delivered to the patient 48 (e.g., because of a mechanical issue or drug diversion).

In some implementations, the patient-controlled drug-requesting device 306 can be used to identify the patient 48 by way of a credential-receiving mechanism or by way of being associated with the patient 48 (e.g., via an identifier stored by the patient-controlled drug-requesting device 306). In some implementations, the patient 48 provides a credential (e.g., patient identity 316 and/or information validating that the patient 48 corresponds to the patient identity 316) to the infusion control device 300 via a user input to the infusion control device 300 (e.g., integrated touchscreen and interface) or a coupled electronic device that is outside the lockbox 302 (e.g., a biometric sensor of the patient-controlled drug-requesting device 306). According to various implementations, after receiving the credential, the infusion control device 300 transmits the credential and/or the patient identity 316 to the patient care device 12, and the patient care device 12 or another component of the patient care system 100 may authenticate the credential.

In some implementations, the patient 48 may provide image data to a camera associated or integrated with patient-controlled drug-requesting device 306, and the patient care device 12 may authenticate the patient 48 based on the image. In some implementations, a credential is received from a badge scanned by an RFID scanner or another card-reading device of the lockbox control interface 304. In some implementations, scanned data received at the lockbox control interface 304 is provided to the data input device 60 of the patient care device 12 (e.g., as a proxy for data that would be collected at a bar code reader of data input device 60).

In some implementations, after the credential is provided, an indication (e.g., an alert response) is received by the infusion control device 300 indicating whether the patient 48 is authorized to request doses of one or more drugs configured to be requested by the patient-controlled drug-requesting device 306. In some implementations, the patient 48 must provide credentials to other devices of the patient care system 100 besides the patient care device 12 to enable functionality of the patient-controlled drug-requesting device 306. In some implementations, the credential for the patient 48 is provided to the information system server 130 and used to access patient historical information, patient therapies, and/or patient physiological data. In some implementations, information (communications, data) received by the interface unit 14 is provided to the infusion control device 300. For example, the functional module 16 may communicate to the interface unit 14 that a syringe pump comprising the functional module 16 is not working properly, and the interface unit 14 may provide that information to the infusion control device 300 (e.g., through the lockbox control interface 304).

According to various implementations, the infusion control device 300 is configured to automatically identify one or more sensor devices 314 (e.g., ETCO2 sensors, SpO2 sensors, etc.), and one or more drug-delivery apparatuses (e.g., functional modules 16 and 18, which may include infusion pumps, syringes, and/or mechanical components thereof) that are in operable communication with the infusion control device 300 (e.g., via the operable communication connection with the patient care system 100). The infusion control device 300 may identify the one or more sensor devices 314 in response to detecting the patient-controlled drug-requesting device 306; or it may do so at a different time, such as when the infusion control device 300 is connected to the patient care device 12 and/or when the patient 48 requests drug delivery. In some implementations, one or more identified sensor devices 314 are integrated (e.g., embedded, attached, or otherwise disposed) into the patient-controlled drug-requesting device 306. For example, a handle of the patient-controlled drug-requesting device 306 may include a camera sensor configured to receive images of the patient 48 and/or a touch-sensitive surface configured to receive patient inputs. According to various implementations, the infusion control device 300 may use the patient identity 316 to confirm patient identity or otherwise authenticate the patient 48. The patient identity 316 may also be used to retrieve patient physiological data or other data about the patient 48 described herein, which may then be used by the infusion control device 300 to authorize requests for doses of drugs to be delivered. For example, patient data indicating that the patient 48 has particular sensitivities (e.g., is more likely to become unconscious or otherwise unstable from PCA) may be used by the infusion control device 300 as part of selecting a drug-control algorithm 308 and/or to select a patient health threshold for comparison with sensor data to determine whether a delivery-control criterion is satisfied.

Based on identifying sensor devices 314 and drug-delivery apparatuses that are electronically coupled to and in electronic communication with the infusion control device 300, the infusion control device 300 automatically selects a drug-control algorithm 308 for controlling drug delivery (e.g., implementing pause control) from a coupled drug-delivery apparatus. The drug-control algorithm 308 may be used to approve drug requests received by the patient-controlled drug-requesting device 306. In this regard, the drug-control algorithm 308 may perform operations to confirm that the patient is authorized to self-administer a drug from the coupled drug-delivery apparatus and only provide instructions to the drug-delivery apparatus to deliver the drug when approved by the algorithm. As will be described further, authorization may include confirming the identity of the patient as well as confirming that the drug may be delivered safely based on patient physiological data, pharmacokinetic properties of the drug being delivered, and/or a history of drug administrations to the patient. In some implementations, the infusion control device 300 obtains the drug-control algorithm 308 from local storage of the infusion control device 300. In some implementations, the infusion control device 300 is configured to download the drug-control algorithm 308 from a different electronic device, such as the information system server 130.

In some implementations, the infusion control device 300 selects a plurality of drug-control algorithms, and each respective drug-control algorithm of the plurality of drug-control algorithms may be specific to a particular drug-delivery apparatus connected to the device. In this regard, the infusion control device 300 may detect the drug-delivery apparatuses connected to the device 300 and select the appropriate algorithm(s) for each drug-delivery apparatus that is detected. In some implementations, the infusion control device 300 determines the algorithms that are required based on the drugs associated with the patient 48 (e.g., in a patient profile accessed based on the patient's identity), the types of drug-delivery apparatuses connected to the device 300, and/or the sensor data that can be obtained by the infusion control device 300 (e.g., via connected sensors) while the patient 48 is using the patient-controlled drug-requesting device 306. In some implementations, the infusion control device 300 detects that the patient care device 12 includes multiple types of drug-delivery apparatuses.

In an example scenario, the patient care device 12 may include a first type of drug-delivery apparatus such as a syringe configured to be manually depressed to inject a first drug into the patient 48 and a second type of drug-delivery apparatus such as an intravenous infusion pump that is integrally coupled with an electronic actuating system (e.g., including mechanical fingers) for causing doses of a second drug to be delivered to the patient 48. The drug-control algorithm 308 may then be selected based on the two drug-delivery apparatuses detected and identified by the infusion control device 300.

According to various implementations, the patient-controlled drug-requesting device 306 is configured to request delivery of a set of drugs corresponding to a set of patient therapies, which may be received by the infusion control device 300 from the information system server 130. In some implementations, power can be reduced or increased to one or more sensor devices 314 of the patient care system 100 based on the set of drug therapies configured to be delivered by connected drug-delivery apparatuses. In some implementations, when a set of therapies is determined, the infusion control device 300 may determine that a particular sensor device 314 (e.g., an ETCO2 sensor device) is required to obtain patient physiological data to determine whether requests for drugs corresponding to the set of therapies should be approved and/or that a biometric verification performed by an imaging sensor of the patient-controlled drug-requesting device 306 is required to access at least one of the drugs associated with the set of therapies. The infusion control device 300 may then confirm that the particular sensor device 314 is connected and, if not connected, prompt a clinician to connect the sensor device 314 and reject requests for drugs until the sensor device 314 is connected and the data received from the sensor device 314 can be assessed by the algorithm. In some implementations, the infusion control device 300 may cause power to be delivered to an identified sensor device 314 (e.g., an ETCO2) required for a given therapy and may reduce power to another sensor device 314 that is not required for implementing the therapy.

According to various implementations of this disclosure, when the patient-controlled drug-requesting device 306 receives a request for a drug to be delivered, the drug-control algorithm 308 of the infusion control device 300 receives an indication of the request and determines whether the request for the drug is authorized by applying sensor data from one or more of the sensor devices 314 that are in operable communication with the infusion control device 300. In some implementations, the determination that the patient 48 is authorized to perform the drug request is based on a biometric verification that the patient 48 actually performed the user input causing the drug request (e.g., to prevent drug diversion). For example, the patient-controlled drug-requesting device 306 may include a camera, and the infusion control device 300 may cause imaging data to be obtained by the camera of the patient-controlled drug-requesting device 306 in response to detecting the request. The obtained image data may be used (e.g., by the patient care device 12 and/or the information system server 130) to perform facial recognition of the patient 48 to verify that the patient 48 performed the input at the patient-controlled drug-requesting device 306.

As will be discussed in greater detail below, the sensor data may be used to determine whether one or more delivery-control criteria are satisfied (e.g., met) before delivering a dose of the drug to the patient 48. In some implementations, delivery-control criteria are a set of one or more prerequisite conditions (e.g., of the patient care system 100 and/or the patient 48) that must be met before a drug-delivery request by the patient 48 is authorized. Additionally, or in the alternative, the delivery-control criteria may be used to determine how much of the drug to deliver, and/or a timing for making delivery of a dose of the drug available to the patient 48. In some implementations, the delivery-control criteria are implemented as part of the drug-control algorithm 308.

The delivery-control criteria can include a threshold medical condition or state (e.g., whether the patient 48 is unstable and/or unconscious) at which the patient 48 is determined to be before the patient 48 can receive a medication. Relevant aspects of the patient's current medical condition or state (e.g., values of biological indicators) may be determined based on measurements from the one or more sensor devices 314 identified by the infusion control device 300, and/or patient data obtained via a different component of the patient care system 100. In one example, a delivery-control criterion can be satisfied based on a biological indicator detected by a sensor device 314 satisfying a patient health threshold for the respective biological indicator, where the biological indicator reflects an aspect of the patient's medical condition or state.

In some implementations, a patient health threshold is set and/or adjusted based on patient data indicating that the patient 48 has a pre-existing medical condition and/or sensitivity to particular types of drugs. A patient health threshold or other delivery-control criterion may further relate to the type of drug being requested and/or pharmacokinetic properties of the drug, which may be identified by the infusion control device 300 as part of detecting the drug-delivery apparatuses of the patient care device 12. For example, determining whether a delivery-control criterion is satisfied may include determining an estimated effect that the drug is likely to have on the patient 48 based on a dose requested by the patient 48, patient demographics, and the pharmacokinetic properties of the drug and, based on the estimated effect, determining whether the delivery of the drug to the patient 48 would cause a biological indicator of the patient to meet or exceed a patient health threshold.

Additionally, or in the alternative, satisfaction of one or more delivery-control criteria may be based on other aspects of the patient care system 100. In some implementations, determining whether a delivery-control criterion is satisfied may include first determining a mechanical state of one of the drug-delivery apparatuses of the patient care device 12. The delivery-control criterion may be satisfied based on determining that mechanical components of a drug-delivery apparatus are properly functioning and capable of delivering a dose of a drug to the patient 48. In some implementations, a first delivery-control criterion related to mechanical components is used by the infusion control device 300 when it is determined that the patient 48 is requesting a drug to be delivered by a first type of drug-delivery apparatus (e.g., a syringe pump), and a second delivery-control criterion related to the mechanical components is used by the infusion control device 300 when it is determined that the patient 48 is requesting a drug to be delivered by a second type of drug-delivery apparatus (e.g., a fluid infusion pump).

In some implementations, satisfaction of a delivery-control criterion may include identifying a mechanical state of a drug-delivery apparatus and selecting a patient health threshold based on the mechanical state. For example, in determining whether a particular delivery-control criterion is satisfied, a biological indicator of the patient 48 may be compared against a first patient health threshold when a drug is being requested from a first type of drug-delivery apparatus, and the same biological indicator of the patient 48 may be compared against a second patient health threshold when the same type of drug is being requested from a second type of drug-delivery apparatus. In some implementations, the selection may be, additionally or in the alternative, based on precision and/or accuracy of the respective drug-delivery-apparatus types in delivering dosage amounts. Satisfaction of drug-delivery criteria may be based on a biometric verification of the patient identity 316 and/or other data collected about the patient 48 besides biological indicators (e.g., historical data about previous deliveries of the drug to the patient 48, such as a pain assessment performed after a previous dose of the drug was delivered to the patient 48). For example, after the patient 48 provides (e.g., via the lockbox 302) a credential to the patient care device 12 as part of determining the patient identity 316, subsequent requests by the patient 48 for delivery of doses of the drug may require additional credentials and/or biometric indicators to verify the requester (e.g., patient 48) as corresponding to the patient identity 316. Satisfaction of a delivery-control criterion may be based on verifying that additional biometric data of the patient 48 corresponds to the patient identity 316. In another example, satisfaction of a delivery-control criterion may be based on verifying that a pain assessment performed by the patient 48 after a prior dose was consistent with assessments done after one or more further doses.

After the infusion control device 300 determines whether the requested dose is authorized for delivery to the patient 48, the infusion control device 300 communicates with the patient care device 12. Such communication may be performed via the lockbox control interface 304, in accordance with some implementations. In some implementations, the infusion control device 300 may communicate directly to the pump control interface 318, which is an interface internal to the patient care device 12, that the patient 48 is not authorized to receive the requested dose, in addition or alternatively to communicating the failed request to the patient care device 12. Communication by the infusion control device 300 with the pump control interface 318 may include instructions for implementing pause control at the drug-delivery apparatus corresponding to the functional module 16.

FIGS. 3B to 3E illustrate example configurations of the infusion control device 300. As described herein, the components (e.g., body 334, sub-assemblies 332-A and 332-B, etc.) of the infusion control device 300 are modular and can be reoriented with respect to each other to achieve different layouts. One of skill in the art will appreciate that the example configurations described with respect to FIGS. 3A to 3E are not the only configurations and/or layouts that the infusion control device 300 may be used in, and that additional configurations and/or layouts not illustrated herein may also be realized. In accordance with the infusion control device 300 having the modular capability to be arranged in the distinct configurations set forth below, the infusion control device 300 provides technical improvements by affording flexibility and adaptability to accommodate different sets of electronic devices that may be required to provide care to a patient 48. According to various implementations, the infusion control device 300 may electronically couple to an interface unit 14 of a patient care device 12, which may include proprietary software distinct from the default software of the infusion control device 300. The infusion control device 300 may be configured to identify aspects of the interface unit 14 and its connected functional modules for control and or interaction with a drug-control algorithm 308 utilized by the interface unit 14.

FIG. 3B illustrates a first example configuration of the infusion control device 300. The infusion control device 300 has a body 334, which includes the display module 310. In accordance with some implementations, the infusion control device 300 includes a plurality of universal connectors (e.g., universal coupling receptacles 330-1, 330-2, and 330-3 (collectively coupling receptacles 330) shown on a top side of the device). In some implementations, each of the universal coupling receptacles 330-1 and 330-2 on the body 334 of the infusion control device 300 is configured to couple with a different type of electronic component, including one or more sub-assemblies of the infusion control device 300. For example, a first type of sub-assembly, a sub-assembly 332-A for housing the drug-requesting device 306 may be coupled to a universal coupling receptacle 330-1 or 330-2 disposed on a right side of the body 334 and a second type of sub-assembly, a sub-assembly 332-B, may be coupled to a universal coupling receptacle 330-1 or 330-2 on a left side of the body 334 of the infusion control device 300.

Each of the sub-assemblies 332-A and 332-B includes a corresponding one or more connectors (e.g., the connector 336-1), which are configured to couple to coupling receptacles 330-1 and 330-2 of the body 334 of the infusion control device 300, and/or other electronic components that are configured to be used in conjunction with the infusion control device 300 (e.g., the lockbox 302, the lockbox control interface 304, and/or the interface unit 14). In accordance with some implementations, when a coupling receptacle 330-1 or 330-2 of the body 334 of the infusion control device 300 becomes coupled with a sub-assembly of the infusion control device 300 or another electronic device (e.g., the interface unit 14 and/or the patient care device 12, the lockbox 302), the infusion control device 300 identifies one or more drug-control algorithms 308 and/or patient care software operations that are relevant to the electronic device.

For example, in accordance with the sub-assembly 332-A (comprising the patient-controlled drug-requesting device 306) becoming coupled with the universal coupling receptacle 330-1 or 330-2 on a side of the body 334, the infusion control device 300 may automatically identify one or more drug-control algorithms 308 to implement that are specific to patient-controlled analgesia that is deliverable via drug requests from the patient-controlled drug-requesting device 306. According to various implementations, when the infusion control device 300 is connected to a specific drug-requesting device 306 and a patient care device 12 (e.g., to interface unit 14), the control device may automatically identify a drug-control algorithm 308 for controlling drug delivery (e.g., initiating a bolus) from the patient care device 12 using the specific patient-controlled drug-requesting device 306.

FIG. 3C illustrates a second example configuration of the infusion control device 300, which includes the same sub-assemblies 332-A and 332-B shown in FIG. 3B. The sub-assemblies 332-A and 332-B are coupled to different universal coupling receptacles 330-1 and 330-2 (e.g., that were not coupled to any sub-assemblies in the first example configuration illustrated in FIG. 3B). Accordingly, the infusion control device 300 recognizes the coupled modules in the same manner as in the configuration of FIG. 3B (or another arrangement) and becomes capable of performing in the same manner, as described above. In the second example configuration, the universal coupling receptacle 330-3 is uncoupled from any sub-assemblies. In some implementations, while the coupling receptacle 330-3 is uncoupled from either of the sub-assemblies 332-A and 332-B, the coupling receptacle 330-3 can be coupled to one or more of: (i) a different sub-assembly comprising a different electronic component configured to be used with the infusion control device 300, (ii) a connector of the patient care device 12, and/or (iii) a connector of the lockbox 302 and/or the lockbox control interface 304.

As shown in FIG. 3C, the display module 310 can be configured in a vertical or horizontal fashion. In some implementations, the infusion control device 300 can identify, based on the configuration of connections to the universal coupling receptacles 330, whether user interfaces of the infusion control device 300 should be presented in a display mode corresponding to the vertical configuration (e.g., a portrait display mode) or a display mode corresponding to the horizontal configuration (e.g., a landscape display mode). Thus, the infusion control device 300 may provide efficient and intuitive means for adjusting user interfaces (and corresponding component arrangement) for use of the infusion control device 300 with a variety of infusion devices and modules.

FIG. 3D illustrates a third example configuration of the infusion control device 300, which includes the sub-assemblies 332-A and 332-B shown in FIGS. 3B and 3C, and the infusion control device 300 is coupled to an interface unit 14 of a patient care device 12, which may include a display 6a that is separate from the display module 310 on the body 334 of the infusion control device 300. In accordance with some implementations, the patient care device 12 can be configured to perform any of the previously described software operations in conjunction with the infusion control device 300. In some implementations, the infusion control device 300 is capable of interacting with the proprietary software of the patient care device 12 (e.g., via an electronic handshake with the patient care unit) in order to identify drug-control algorithms 308 or other software operations that are relevant to respective electronic components of the patient care device 12. The infusion control device 300 may identify that the patient care device 12 includes the display 6a that is configured to display user interfaces related to patient care. In accordance with identifying the display 6a of the patient care device 12, the infusion control device 300 may be configured to cause user interfaces to be presented at the display 6a of the patient care device 12 (or cause user interfaces of the patient care device 12 to be presented at the display module 310 on the body 334 of the infusion control device 300). In some implementations, the infusion control device 300 is configured to receive signals from one or more sensor devices 314 or other electronic components that are not physically connected to the infusion control device 300 (e.g., via a Bluetooth connection).

FIG. 3E illustrates a fourth example configuration of the infusion control device 300, in which the body 334 and or the sub-assemblies 332-A and 332-B are coupled to a hardware shelf 350 of the lockbox 302, which may be configured with further corresponding coupling receptacles 330 (shown on FIG. 3C) for electronically coupling the infusion control device 300 to the lockbox control interface 304. In some implementations, the hardware shelf 350 may house wires for coupling the coupling receptacles 330 (not shown) provided by the shelf to the lockbox 302 and/or the patient care unit that is housed within the lockbox 302. In some implementations, the lockbox control interface 304 forms a part of, or is disposed on, a lower surface of the hardware shelf 350. In some implementations, in response to the infusion control device 300 being coupled to the lockbox 302 (e.g., via the hardware shelf 350), the display module 310 displays one or more authentication user interfaces for providing patient and/or care provider credentials to the lockbox control interface 304. In some implementations, while the infusion control device 300 is coupled with the lockbox 302 and/or the lockbox control interface 304, and the infusion control device 300 includes the sub-assembly 332-A comprising the drug-requesting device 306, the patient 48 may provide credentials to the lockbox 302 via one or more electronic components of the patient-controlled drug-requesting device 306 (e.g., via a fingerprint scan, an iris scan, and/or facial recognition enabled by one or more imaging sensors located on the patient-controlled drug-requesting device 306). In some embodiments, door access to the lockbox 302 (e.g., to add, remove, or change medications of the patient care device 12) can be activated directly through user interfaces of the infusion control device 300 (e.g., without requiring a physical lock and key).

Accordingly, in some implementations, the universal connection receptacles 330 of the infusion control device 300 allow for forming of a unitary functional arrangement of components that includes the patient care device 12, the lockbox 302, and the infusion control device 300, such that any pump and/or syringe that is provided to the patient care device 12 can be used in conjunction with one or more drug-control algorithms 308 accessible by the infusion control device 300. In some implementations, while the infusion control device 300 is coupled with the lockbox 302, settings of the patient care device 12 can be configured using the infusion control device 300, without requiring opening of the door of the lockbox 302. In some embodiments, the infusion control device 300 is configured to identify one or more sensors of the lockbox 302 (e.g., a camera located on the lockbox 302).

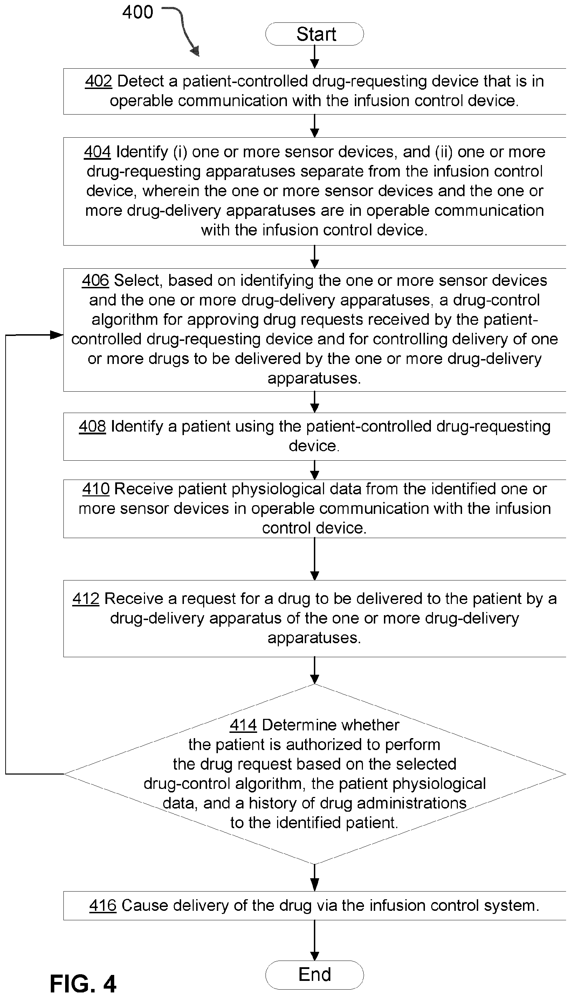

FIG. 4 depicts an example process for implementing a drug-control algorithm 308 at an infusion control device 300 in operable communication with one or more drug-delivery apparatuses, according to various aspects of the subject technology. For explanatory purposes, the various blocks of example process 400 are described herein with reference to FIGS. 1-3, and the associated components and/or processes described herein. The one or more blocks of example process 400 may be implemented, for example, by one or more computing devices including, for example, interface unit 14, information system server 130, one or more of functional modules 16, 18, 20, and 22, infusion control device 300, and/or a client computing device. In some implementations, one or more of the blocks may be implemented using machine-learning algorithms. In some implementations, one or more of the blocks may be implemented apart from other blocks, and by one or more different processors or devices. Further, for explanatory purposes, to the extent that the blocks of the example process 400 are described as occurring serially, or linearly, in some implementations, multiple blocks of the example process 400 may occur in parallel (e.g., blocks 406 and 408 may occur in parallel, and any of blocks 404, 406, and 408 may occur after the patient 48 requests delivery of the dose as discussed in block 410). The blocks of the example process 400 need not be performed in the order shown, and one or more blocks of the example process 400 need not be performed, in accordance with some implementations.

In the depicted example, the blocks of the example process 400 are performed by the infusion control device 300. The infusion control device 300 detects that the patient-controlled drug-requesting device 306 is in operable communication with the infusion control device 300 (as described in block 402). In some implementations, the patient-controlled drug-requesting device 306 is integrally attached to the infusion control device 300. In some implementations, the patient-controlled drug-requesting device 306 is physically separate from the infusion control device 300 but electronically coupled to the infusion control device 300 via a wired or wireless connection. In some implementations, the infusion control device 300 is configured to detect and couple with a patient care device 12, and then detect the patient-controlled drug-requesting device 306 after detachably coupling with patient care device 12 (and/or otherwise forming an electronic communication connection with the patient care system 100). For example, infusion control device 300 may detachably couple to lockbox 302 (e.g., via the lockbox control interface 304), which may be enclosing the patient care device 12.

The infusion control device 300 identifies, based on detecting the patient-controlled drug-requesting device 306, one or more sensor devices 314 and one or more drug-delivery apparatuses separate from the infusion control device 300, wherein the one or more sensor devices 314 and the one or more drug-delivery apparatuses are in operable communication with the infusion control device 300 (as described in block 404). For example, the infusion control device 300 may detect one or more functional modules 16, 18, 20, and 22 of the patient care device 12. In some implementations, the infusion control device 300 detects which drugs each respective drug-delivery apparatus is delivering or is programmed to deliver to the patient 48, and which of the respective drugs are available to be requested by the patient-controlled drug-requesting device 306. The infusion control device 300 may detect sensor devices 314 of the patient-controlled drug-requesting device 306 (e.g., a camera sensor, a touch-sensitive surface, an embedded grip sensor for detecting a health parameter) and/or other sensor devices 314 (e.g., ETC02, SPO2, etc.) operably connected to the patient care device 12. In some implementations, when the infusion control device 300 detects the one or more drug-delivery apparatuses, the infusion control device 300 identifies one or more of fluid administration sets 30, 32, 34, and 36 and/or fluid supplies 38, 40, 42, and 44. In some implementations, the infusion control device 300 selects the drug-control algorithm 308 based on a respective fluid administration set of fluid supply of the one or more drug-delivery apparatuses. In some implementations, infusion control device 300 is configured to detect operating parameters of one or more fluid infusion pumps and provide a user input (e.g., a menu-driven graphical user interface) for viewing and/or changing the detected operating parameters.

Based on identifying the one or more sensor devices 314, patient physiological data received from the sensors, operating parameters, and/or the one or more drug-delivery apparatuses, the infusion control device 300 selects a drug-control algorithm 308 for approving drug requests received by the patient-controlled drug-requesting device 306 (as described in block 406). The infusion control device 300 may select the drug-control algorithm 308 from a predefined set of drug-control algorithms 308 that the infusion control device 300 stores locally in a memory of the infusion control device 300. For example, the infusion control device 300 may store a particular set of drug-control algorithms 308 for a particular set of default therapies that the infusion control device 300 can implement automatically, without further instruction from the patient 48.

According to some implementations, when the infusion control device 300 selects a drug-control algorithm 308 that is not locally stored, the infusion control device 300 may download the selected drug-control algorithm 308 (e.g., information system server 130) from a server that is in operable communication with the patient care system 100 and/or the infusion control device 300. In some implementations, the infusion control device 300 may select the drug-control algorithm 308 from a library available at the device network 140 of the healthcare network 110. In some implementations, the infusion control device 300 may identify a locally stored drug-control algorithm 308 to implement, and may also identify another drug-control algorithm 308 that is not stored locally on the infusion control device 300 that would be more effective for the patient 48 (e.g., based on patient physiological data) and/or more efficient to implement based on the one or more sensor devices 314 detected by the infusion control device 300. When the infusion control device 300 identifies the other drug-control algorithm 308 that is not locally stored but that is more effective than a locally stored algorithm, the infusion control device 300 may implement the locally stored drug-control algorithm 308 temporarily until the other drug-control algorithm 308 is downloaded, installed, or otherwise made available to the infusion control device 300.

The infusion control device selects the drug-control algorithm 308 for monitoring and controlling drug-delivery apparatuses based on detecting connected devices. For example, when a respective drug-delivery apparatus includes a syringe (e.g., as part of an integrated syringe pump 18), the infusion control device 300 selects a first algorithm specific to the syringe, and when the respective drug-delivery apparatus is a large-volume pump, the infusion control device 300 selects a second algorithm specific to large-volume pumps. Additionally, or in the alternative, the drug-control algorithm 308 may be selected based on detecting a particular sensor device 314 or set of sensor devices. For example, a first drug-control algorithm that uses a first delivery-control criterion may be used based on detecting a first type of sensor device 314 (e.g., an ETCO2 sensor) in operable communication with the infusion control device 300, and a second drug-control algorithm that uses a second delivery-control criterion may be used based on detecting a second type of sensor device 314 (e.g., a heart-rate monitor) in operable communication with the infusion control device 300. In some implementations, a drug-control algorithm 308 may be selected based on determining which sensor device(s) of a plurality of sensor devices 314 detected by the infusion control device 300 would be more accurate. For example, the infusion control device 300 may detect that there are two types of sensor devices 314 in operable communication with the patient care device 12 that are capable of detecting whether the patient 48 is in a stable condition (e.g., an ETCO2 sensor device, and a heart-rate sensor device). Based on a determination that a particular sensor device 314 of the plurality of sensor devices is more accurate for detecting a medical condition and/or state of the patient 48, the infusion control device 300 may select a respective drug-control algorithm 308 that uses data from the particular sensor device.

According to some implementations, the infusion control device 300 can monitor software associated with the patient control device 12 and/or module-specific components 76 of the functional modules 16, 18, 20, and 22. In some implementations, when the infusion control device 300 determines that the software or a module-specific component 76 is already implementing pause control at a respective functional module, the infusion control device 300 can determine to either pass patient requests through to the PCD software or module-specific component 76, or to deactivate and/or override the pause control being implemented by the PCD software or module-specific component 76. In some implementations, as part of performing operations of a selected drug-control algorithm 308, the infusion control device 300 may provide minimum and/or maximum values for ETCO2, respiration rate, and/or other monitored parameters. Such minimum and/or maximum values may be set in addition or alternatively to values set at the interface unit 14 by a clinician. These minimum and/or maximum values may be used by the infusion control device 300 (e.g., as delivery-control criteria) to determine whether to pass patient requests through to the PCD software or module-specific component 76, or to deactivate and/or override the pause control being implemented by the PCD software or module-specific component 76.

The infusion control device 300 identifies the patient 48 using the patient-controlled drug-requesting device 306 (as described in block 408). In some implementations, the patient-controlled drug-requesting device 306 obtains a credential from the patient 48, which may then be forwarded to and used by the PCD 12 to authenticate the patient (e.g., by determining the patient identity 316). In some implementations, the credential is obtained as biometric data using a sensor device of the one or more sensor devices identified by the infusion control device 300. For example, the infusion control device 300 may detect that the patient-controlled drug-requesting device 306 includes a fingerprint scanner, and, based on detecting the fingerprint scanner, the infusion control device 300 may cause the patient-controlled drug-requesting device 306 to instruct the patient 48 to perform a fingerprint scan and validate the identity of the patient 48 based on the obtained fingerprint. In some implementations, after identifying the patient identity 316, the infusion control device 300 may subsequently identify and/or verify the patient 48 (e.g., as part of determining that a particular drug request made by the patient 48 is authorized) by querying a patient records system or database (e.g., at information system server 130).

The infusion control device 300 receives patient physiological data from the one or more sensor devices in operable communication with the infusion control device 300 (as described in block 410). For example, the one or more sensor devices may include an SPO2 sensor device configured to measure the amount of oxygen in the blood of the patient, which can be used to determine whether the patient 48 is at risk of having low oxygen levels indicative of a particular medical condition or state (e.g., becoming unconscious or otherwise unstable as a result of being over-sedated by the drug the patient 48 is requesting delivery of, or another drug that affects related patient physiological data). The patient physiological data may be applied to the drug-control algorithm 308 to determine whether a drug request performed by the patient 48 is authorized and/or for other operations performed by the infusion control device 300 as part of the example method 400. For example, the patient physiological data may include a biological indicator that is used by the drug-control algorithm 308 to determine if one or more delivery-control criteria are satisfied for authorizing a drug request performed by the patient 48. In some implementations, the patient physiological data may be used by the infusion control device 300 to determine which respective drugs can be requested by the patient 48 via the patient-controlled drug-requesting device 306 (e.g., based on a stored list of patient therapies associated with the patient 48 and/or physiological data thresholds or values).

The infusion control device 300 receives a request for a drug to be delivered to the patient by a drug-delivery apparatus of the one or more drug-delivery apparatuses (as described in block 412).

In some implementations, the patient requests delivery of the drug by activating a control on the patient-controlled drug-requesting device 306 (e.g., a selectable portion of the display 320 of the drug-requesting device 306). In some implementations, the patient can request delivery of the drug by selecting a user interface element shown within the display 320 of the patient-controlled drug-requesting device 306. In some implementations, additional information is collected while the patient 48 is requesting the delivery of the dose of the drug (e.g., information to verify the identity of the patient and/or patient physiological data that is relevant to the request). In some implementations, one or more sensor devices of the patient-controlled drug-requesting device 306 begin collecting the identity or physiological data when the patient 48 initiates the request for the drug to be delivered, and the selected drug-control algorithm determines whether the request is authorized based on the received data.

Responsive to receiving the request for the drug to be delivered to the patient 48, the selected drug-control algorithm 308 of the infusion control device 300 determines whether the patient 48 is authorized to perform the drug request based on the patient physiological data, and a history of drug administrations to the identified patient (as described in block 414). According to some implementations, determining whether the patient 48 is authorized to perform the request is based on comparing sensor data collected by a sensor device to one or more patient health thresholds (e.g., comparing a value of an exhaled concentration of CO₂ detected by an ETCO2 sensor device of the one or more sensor devices to a threshold value indicating that the patient 48 is or is likely to become unconscious or otherwise unstable).

According to some implementations, in determining whether the patient 48 is authorized to perform the request for the drug, the infusion control device 300 receives data for determining whether one or more delivery-control criteria are satisfied. For example, satisfaction of a delivery-control criterion may be based on comparing a biological indicator of the patient 48 to a patient health threshold (e.g., a guardrail), where the patient health threshold is configured to identify whether the patient 48 is unstable, unconscious, or otherwise impaired by delivery of the drug, and/or whether the delivery of the drug could cause such a health condition of the patient 48 to be present. In some implementations, whether delivery of the drug could cause a biological indicator to meet or exceed a respective patient health threshold may be based on pharmacokinetic properties of a drug being requested (e.g., by estimating an effect on a respective biological indicator of the patient 48 based on the pharmacokinetic properties. For example, the infusion control device 300 may determine that pharmacokinetic properties of a drug being requested would cause a value of a measured biological indicator of the patient 48 to change (e.g., an estimated effect) such that it could cause the patient 48 to become unstable. Based on the determination, the drug-control algorithm 308 may prevent delivery of a requested drug or implement a patient health threshold with a minimum or maximum value that accounts for the potential change in value of the biological indicator.

In some implementations, alternative patient health thresholds can be implemented for respective biological indicators based on the sensor devices being used to detect the values of the biological indicators (e.g., based on a sensitivity of a respective sensor device, or a type of biological indicator that the one or more sensor devices are configured to detect). For example, a first patient health threshold for a biological indicator (e.g., a concentration of CO₂ being exhaled by the patient 48) may be used if the one or more sensor devices include a first type of ETCO2 sensor (e.g., a sidestream sensor), and a second patient health threshold may be used if the one or more sensor devices include a second type of ETCO2 sensor (e.g., a mainstream sensor). That is, the second patient health threshold may be closer to a value indicating a critical condition than the first patient health threshold, based on the increased sensitivity and/or continuous nature of monitoring of the second type of ETCO2 sensor.

According to some implementations, one or more additional delivery-control criteria may be used to determine whether the patient 48 is authorized to request delivery of the drug. In some implementations, the infusion control device 300 may receive information about a pharmacokinetic property of the drug being requested by the patient 48, a biometric indicator detected via the patient-controlled drug-requesting device 306 when the patient 48 requests delivery of the drug (e.g., image data from a camera, data from an electronic fingerprint scanner embedded in the handle of the patient-controlled drug-requesting device 306), and/or another type of patient input provided to the patient-controlled drug-requesting device 306. For example, after a previous dose has been provided to the patient 48, a pain assessment user interface may be presented at the display module 310 associated with the patient-controlled drug-requesting device 306, and the patient may be prompted to provide input. The input provided to the pain assessment user interface by the patient 48 may be used to determine whether the requested delivery to the patient 48 is authorized.

In some implementations, the number of previous drug-delivery requests by the patient 48 (e.g., within a predetermined period) can be used to determine whether delivery-control criteria are satisfied for a current drug request by the patient 48. For example, the patient 48 may be allowed three dose units of a drug per hour. The patient 48 may be allowed to select the number of dose units to receive but not more than the set maximum. In another example, a patient health threshold may be set based on whether one or more previous deliveries of a drug caused the patient 48 to become over-sedated and/or were ineffective in reducing pain for the patient 48. That is, a first patient health threshold may be used by default (e.g., a predefined patient health threshold) and a second patient health threshold may be used in place of the first patient health threshold based on the patient 48 having a negative reaction to a previous dose (e.g., while a biological indicator of the patient 48 was below the first patient health threshold). As another example, patient physiological data from a previous request by the patient 48 may indicate that, shortly after receiving delivery of a requested drug, the patient 48 performed a pain assessment and indicated that they were still experiencing an elevated level of pain, which may indicate that delivery of the previous dose of the drug was ineffective (e.g., as a result of the drug being diverted from the patient 48 or otherwise failing to be delivered properly). Based on the patient data related to the pain assessment performed after the previous drug request, satisfaction of an additional delivery-control criterion may be required to authorize the current drug request, where satisfying the additional delivery-control criterion requires a verification (e.g., based on biometric data) that the patient 48 actually initiated the drug request (e.g., the patient-controlled drug-requesting device 306 may prompt the user to provide image or fingerprint data to verify that the patient 48 corresponds to the patient identity 316).

According to some implementations, one or more delivery-control criteria may be apparatus-dependent (e.g., dynamically adjustable) based on the type of drug-delivery apparatus identified for delivering a dose of a drug to the patient 48. For example, there may be a first drug-control threshold for a particular patient health parameter associated with delivery of a particular drug via a first type of drug-delivery apparatus (e.g., a syringe pump) and a second drug-control threshold for the same particular patient health parameter associated with delivery of the particular drug via a second type of drug-delivery apparatus (e.g., an intravenous infusion pump). Additionally, or in the alternative, the different drug-control thresholds may be based on respective levels of control that are available by the respective pump types.

In some implementations, one or more pieces of the patient physiological data are provided to a machine-learning model. The machine-learning model may be trained with patient-specific data of the patient 48 (e.g., historical drug-delivery data), and/or with patient data for a patient population. In this regard, the machine-learning model may map outcomes to the patient physiological data (e.g., based on patient demographics, drugs, and/or drug dosages). The infusion control device 300 may receive a drug-request authorization indication from the machine-learning model based on a mapped outcome for the patient physiological data received by the machine-learning model. In some implementations, the machine-learning model may indicate other information related to the drug request. For example, the machine-learning model may indicate an amount of time before a drug request submitted by the patient 48 is estimated to meet delivery-control criteria (e.g., based on the respective values of patient physiological data over a particular period). In some implementations, the indication from the machine-learning model is provided to the infusion control device 300, which may, responsive to receiving the indication, cause drug delivery to be temporarily restricted by one or more drug-delivery apparatuses of the patient care device 12. In some implementations, based on the machine-learning model indicating the amount of time before a drug request submitted by the patient 48 is estimated to meet delivery-control criteria, the infusion control device 300 may cause the patient-controlled drug-requesting device 306 to forego presenting any selectable elements to the patient 48 for requesting delivery of the drug. In some implementations, based on an amount by which a particular delivery-control criterion is not satisfied, the display 320 of the patient-controlled drug-requesting device 306 may display a time-based indication (e.g., a countdown timer) indicating when delivery of the drug will be available (e.g., based on when a particular condition causing the delivery-control criteria to not be satisfied will cease to be present).

Based on determining that the patient 48 is authorized to perform the drug request, the infusion control device 300 causes delivery of the drug via the one or more drug-delivery apparatuses (as described in block 416). According to some implementations, the infusion control device 300 is configured to cause delivery of drugs via multiple types of drug-delivery apparatuses, and the infusion control device 300 detects the type of drug-delivery apparatus that is being used to deliver the drug requested by the patient 48. In some implementations, the infusion control device 300 provides information to the interface unit 14, and the interface unit 14 may translate the information into programming instructions that the interface unit 14 is configured to provide to functional modules 16, 18, 20, and 22. In this regard, the infusion control device 300 may instruct the interface unit 14 on how to control its connected modules. For example, the infusion control device 300 may provide flow-control instructions to a respective fluid infusion pump of the one or more drug-delivery apparatuses. In some implementations, the infusion control device 300 is configured to provide instructions to the interface unit 14 as instruction-based inputs to the control keys 6b and/or 6c.

In some implementations, after the drug-control algorithm 308 determines that the patient 48 is not authorized to receive a dose of the requested drug, the infusion control device 300 prevents the patient 48 from receiving delivery of the drug for a predetermined amount of time (e.g., a pause-control duration). In some implementations, the infusion control device 300 presents an indication (e.g., a notification user interface element) to the patient 48 indicating that there was an error authorizing the drug request and/or additional drug-delivery information or that the request was not authorized. For example, the display 320 of the patient-controlled drug-requesting device 306 may present an indication about one or more aspects of the patient physiological data that was relevant to the drug request authorization being denied, and/or an amount of time until the patient 48 will be able to request delivery of the drug.

In some implementations, one or more drug-delivery criteria may not be met for delivery of one drug, but the same patient physiological data may be used to determine that delivery of a different drug is authorized. For example, while the patient 48 is prevented from receiving delivery of a first drug for the predetermined amount of time (e.g., based on values of one or more delivery-control criteria being beyond a particular threshold), the infusion control device 300 may determine that a second drug different from the first drug would be authorized to be delivered to the patient 48 by another drug-delivery apparatus associated with (e.g., connected to) the infusion control device 300. Based on the same or similar patient physiological data that failed to meet (e.g., satisfy) delivery-control criteria for authorizing the first drug request, the infusion control device 300 may determine that the patient 48 is authorized to receive the dose of the second drug. That is, there may be a different guardrail for a particular health parameter with respect to delivery of the second drug than for delivery of the first drug. In some implementations, when the request for the first drug is not authorized, the patient care system 100 may prompt the patient 48 (e.g., via a display on the drug delivery device), asking whether the patient 48 would like to receive the second drug. The patient 48 may then confirm selection of the second drug via the patient-controlled drug-requesting device 306 (e.g., by activating a control on the device or via touchscreen input on the display 320 of the patient-controlled drug-requesting device 306).

In some implementations, after the drug-control algorithm has been selected, the infusion control device 300 is configured to detect a different device forming an electronic communication connection with the infusion control device 300 (e.g., an external sensor), or to a different electronic device of the patient care system 100. In some implementations, after detecting the different device forming the electronic connection, the infusion control device 300 identifies a second drug-control algorithm for authorizing requests for doses of the drug to the patient 48, the second drug-control algorithm associated with the different device and distinct from the drug-control algorithm 308 (e.g., the first drug-control algorithm). After identifying the second drug-control algorithm, the infusion control device 300 may replace the first drug-control algorithm with the second drug-control algorithm. That is, the infusion control device 300 can be configured to dynamically optimize drug control by switching which drug-control algorithm 308 is being used when it detects a change to the composition of the patient care system 100.

Many of the above-described operations of example process 400, and related features and applications, may also be implemented as software processes that are specified as a set of instructions recorded on a computer-readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, and so forth. "Computer readable media" does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

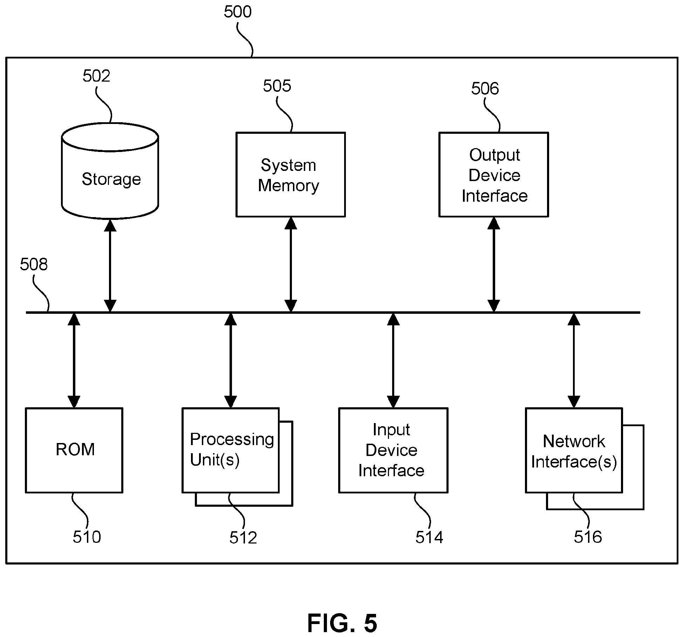

FIG. 5 is a conceptual diagram illustrating an example electronic system 500 for operating an analgesia administration system, according to aspects of the subject technology. Electronic system 500 may be a computing device for execution of software associated with one or more portions or steps of process 400 of FIG. 1, or components and processes provided by FIGS. 1-3, including but not limited to information system server 130, computing hardware within patient care device 12, or administration set 32. Electronic system 500 may be representative, in combination with the disclosure regarding FIGS. 1-4. In this regard, electronic system 500 may be a personal computer or a mobile device such as a smartphone, tablet computer, laptop, PDA, an augmented reality device, a wearable such as a watch or band or glasses, or combination thereof, or other touchscreen or television with one or more processors embedded therein or coupled thereto, or any other sort of computer-related electronic device having network connectivity.

Electronic system 500 may include various types of computer readable media and interfaces for various other types of computer readable media. In the depicted example, electronic system 500 includes a bus 508, processing unit(s) 512, a system memory 504, a read-only memory (ROM) 510, a permanent storage device 502, an input device interface 514, an output device interface 506, and one or more network interfaces 516. In some implementations, electronic system 500 may include or be integrated with other computing devices or circuitry for operation of the various components and processes previously described.

Bus 508 collectively represents all system, peripheral, and chipset buses that communicatively connect the numerous internal devices of electronic system 500. For instance, bus 508 communicatively connects processing unit(s) 512 with ROM 510, system memory 504, and permanent storage device 502.

From these various memory units, processing unit(s) 512 retrieves instructions to execute and data to process in order to execute the processes of the subject disclosure. The processing unit(s) can be a single processor or a multi-core processor in different implementations.

ROM 510 stores static data and instructions that are needed by processing unit(s) 512 and other modules of the electronic system. Permanent storage device 502, on the other hand, is a read-and-write memory device. This device is a non-volatile memory unit that stores instructions and data even when electronic system 500 is off. Some implementations of the subject disclosure use a mass-storage device (such as a magnetic or optical disk and its corresponding disk drive) as permanent storage device 502.

Other implementations use a removable storage device (such as a floppy disk, flash drive, and its corresponding disk drive) as permanent storage device 502. Like permanent storage device 502, system memory 504 is a read-and-write memory device. However, unlike storage device 502, system memory 504 is a volatile read-and-write memory, such a random-access memory. System memory 504 stores some of the instructions and data that the processor needs at runtime. In some implementations, the processes of the subject disclosure are stored in system memory 504, permanent storage device 502, and/or ROM 510. From these various memory units, processing unit(s) 512 retrieves instructions to execute and data to process in order to execute the processes of some implementations.

Bus 508 also connects to input and output device interfaces 514 and 506. Input device interface 514 enables the user to communicate information and select commands to the electronic system. Input devices used with input device interface 514 include, e.g., alphanumeric keyboards and pointing devices (also called "cursor control devices"). Output device interfaces 506 enables, e.g., the display of images generated by the electronic system 500. Output devices used with output device interface 506 include, e.g., printers and display devices, such as cathode ray tubes (CRT) or liquid crystal displays (LCD). Some implementations include devices such as a touchscreen that functions as both input and output devices.

Also, as shown in FIG. 5, bus 508 also couples electronic system 500 to a network (not shown) through network interfaces 516. Network interfaces 516 may include, e.g., a wireless access point (e.g., Bluetooth or WiFi) or radio circuitry for connecting to a wireless access point. Network interfaces 516 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 500 can be used in conjunction with the subject disclosure. These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer readable medium (also referred to as computer readable storage media, machine-readable media, or machine-readable storage media). Some examples of such computer readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra-density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer," "server," "processor," and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Implementations of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some implementations, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

Illustration of Subject Technology as Clauses:
Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identifications.

Clause 1. An infusion control device, comprising: one or more processors; and a memory storing instructions which, when executed by the one or more processors, cause the one or more processors to perform operations comprising: detecting a patient-controlled drug-requesting device that is in operable communication with the infusion control device; identifying (i) one or more sensor devices, and (ii) one or more drug-delivery apparatuses separate from the infusion control device, in operable communication with the infusion control device, wherein the one or more sensor devices and the one or more drug-delivery apparatuses are in operable communication with the infusion control device; selecting, based on identifying the one or more sensor devices and the one or more drug-delivery apparatuses, a drug-control algorithm for approving drug requests received by the patient-controlled drug-requesting device and for controlling delivery of one or more drugs to be delivered by the one or more drug-delivery apparatuses; identifying a patient using the patient-controlled drug-requesting device; receiving patient physiological data from the identified one or more sensor devices in operable communication with the infusion control device; receiving a request for a drug to be delivered to the patient by a drug-delivery apparatus of the one or more drug-delivery apparatuses; determining whether the patient is authorized to perform the drug request based on a function of the drug-control algorithm, the patient physiological data, and a history of drug administrations to the patient; and causing, based on determining, using the drug-control algorithm, that the patient is authorized to perform the drug request, delivery of the drug via the one or more drug-delivery apparatuses.

Clause 2. The infusion control device of Clause 1, wherein the determining whether the patient is authorized to perform the request is based on satisfying one or more delivery-control criteria related to one or more of: a determination that a biological indicator of the patient is beyond a patient health threshold for administering the drug; a pharmacokinetic property of the drug being requested; a biometric indicator detected via the patient-controlled drug-requesting device; and a pain assessment performed after a prior delivery of the drug in response to a prior request for the drug.

Clause 3. The infusion control device of Clause 1 or Clause 2, wherein operations further comprise: providing the patient physiological data to a machine-learning model; and receiving a drug-request authorization indication from the machine-learning model, wherein the drug-request authorization indication is used to determine whether the patient is authorized to receive a requested dose of a drug.

Clause 4. The infusion control device of any one of Clauses 1 through 3, wherein the operations further comprise, prior to identifying the one or more drug-delivery apparatuses: forming an electronic communication connection with a lockbox control interface configured to enclose and prevent access to at least a portion of the one or more drug-delivery apparatuses; receiving a wireless credential at an input interface of the infusion control device; transmitting, by the infusion control device, the wireless credential to the one or more drug-delivery apparatuses via the electronic communication connection with the lockbox control interface; receiving an indication from the one or more drug-delivery apparatuses via the electronic communication connection, after the transmitting, whether the patient is authorized to request doses of the drug to be delivered; and in accordance with receiving an indication that the patient is authorized to request doses of the drug to be delivered, using the drug-control algorithm to determine if a drug request submitted by the patient is an authorized drug request.

Clause 5. The infusion control device of any one of Clauses 1 through 4, wherein: detecting the one or more drug-delivery apparatuses comprises: when a respective drug-delivery apparatus is a first type of drug-delivery apparatus including a syringe configured to be manually pressed to inject a first drug into the patient, selecting a first algorithm for delivering the drug via the first type of drug-delivery apparatus as the drug-control algorithm, the first algorithm including instructions for causing the syringe to inject the first drug into the patient; and when the respective drug-delivery apparatus is a second type of drug-delivery apparatus including an intravenous infusion pump that is integrally coupled with an electronic actuating system for causing doses of a second drug to be delivered to the patient, selecting a second algorithm for delivering the drug via the second type of drug-delivery apparatus as the drug-control algorithm, the second algorithm including instructions for causing delivery via the intravenous infusion pump.

Clause 6. The infusion control device of any one of Clauses 1 through 5, wherein the operations further comprise: determining one or more therapies that the patient-controlled drug-requesting device is able to receive drug requests for from the patient; based on determining the one or more therapies enabled by the patient-controlled drug-requesting device, selecting one or more drug-control algorithms for each respective therapy of the one or more therapies; and providing each respective drug-control algorithm of the one or more drug-control algorithms for use in conjunction with the patient-controlled drug-requesting device.

Clause 7. The infusion control device of any one of Clauses 1 through 6, wherein the operations further comprise, after the drug-control algorithm is selected: detecting electronic connection of a different device to the infusion control device; identifying a second drug-control algorithm, associated with the different device and distinct from the drug-control algorithm, for authorizing requests for doses of the drug to the patient; and replacing a selected drug-control algorithm with the second drug-control algorithm.

Clause 8. The infusion control device of any one of Clauses 1 through 7, wherein the drug-control algorithm is configured to prevent the drug delivery of a particular drug while the patient physiological data does not meet one or more predefined delivery-control criteria that are required for authorizing doses of the drug to be delivered.

Clause 9. The infusion control device of Clause 8, wherein the operations further comprise, after preventing the drug delivery of the particular drug based on a determination that a patient is not authorized to perform the request: preventing the patient from receiving delivery of the particular drug for a predetermined amount of time.

Clause 10. The infusion control device of Clause 9, further comprising: while the patient is prevented from receiving delivery of the particular drug for the predetermined amount of time: receiving a second request for another drug, different from the particular drug, to be delivered to the patient by another drug-delivery apparatus of the one or more drug-delivery apparatuses; and based on the patient physiological data that did not meet the one or more delivery-control criteria for authorizing the request, determining that the patient is authorized to perform the second request for the other drug; and causing, based on determining that the patient is authorized to perform the second request, delivery of the other drug via the one or more drug-delivery apparatuses.

Clause 11. The infusion control device of Clause 9, further comprising: after determining that the patient is not authorized to perform the first request, determining that the patient physiological data that did not meet would meet other delivery-control criteria for authorizing a request for delivery of another drug; and based on determining that a request for delivery of the other drug would be authorized based on the patient physiological data, prompting the patient that the other drug is available to be delivered.

Clause 12. The infusion control device of any one of Clauses 1 through 11, wherein the identifying of the one or more sensor devices, and the one or more drug-delivery apparatuses is performed based on detecting that the patient-controlled drug-requesting device is in operable communication with the infusion control device.

Clause 13. The infusion control device of any one of Clauses 1 through 12, further comprising: a body, the body comprising: the display, and one or more universal coupling receptacles configured to receive respective sub-assemblies of the infusion control device, wherein each of a plurality of sub-assemblies is configured to form an operable connection with each of the universal coupling receptacles; one or more sub-assemblies, wherein: each of the one or more sub-assemblies comprise respective constituent electronic components of the infusion control device, each of the one or more sub-assemblies comprise universal connectors configured to operable couple with the one or more universal connection coupling receptacles of the body, wherein, in accordance with a respective universal connector of a respective sub-assembly of the one or more sub-assemblies becoming coupled with a respective connection point of the body, constituent electronic components of the infusion control device become operably coupled with other constituent electronic components of the body.

Clause 14. The infusion control device of Clause 13, wherein: the one or more sub-assemblies include a first sub-assembly of a first type, wherein the first sub-assembly comprises the drug-requesting device, and the one or more sub-assemblies include a second sub-assembly of a second type, wherein the second sub-assembly comprises a sensor device of the one or more sensors devices.

Clause 15. The infusion control device of Clause 13 or Clause 14, wherein each of the one or more universal coupling receptacles is configured to be coupled with one of the following: a patient care unit comprising proprietary software distinct from default software of the infusion control device; a lockbox and/or a lockbox control interface associated with the lockbox, and/or a hardware shelf associated with the lockbox; and a sensor device of the one or more sensor devices, distinct from another sensor device associated with the one or more sub-assemblies.

Clause 16. A method, comprising: at an infusion control device that includes one or more processors and a memory storing instructions: detecting a patient-controlled drug-requesting device that is in operable communication with the infusion control device; identifying, based on detecting the patient-controlled drug-requesting device, (i) one or more sensor devices, and (ii) one or more drug-delivery apparatuses separate from the infusion control device, wherein the one or more sensor devices and the one or more drug-delivery apparatuses are in operable communication with the infusion control device; selecting, based on identifying the one or more sensor devices and the one or more drug-delivery apparatuses, a drug-control algorithm for approving drug requests received by the patient-controlled drug-requesting device; identifying a patient using the patient-controlled drug-requesting device; receiving patient physiological data from the one or more sensor devices in operable communication with the infusion control device; receiving a request for a drug to be delivered to the patient by a drug-delivery apparatus of the one or more drug-delivery apparatuses; determining whether the patient is authorized to perform the drug request based on a function of the drug-control algorithm, the patient physiological data, and a history of drug administrations to the patient; causing, based on determining, using the drug-control algorithm, that the patient is authorized to perform the drug request, delivery of the drug via the one or more drug-delivery apparatuses.

Clause 17. The method of Clause 16, wherein the determining whether the patient is authorized to perform the request is based on satisfying one or more delivery-control criteria related to one or more of: a determination that a biological indicator of the patient is beyond a patient health threshold for administering the drug; a pharmacokinetic property of the drug being requested; a biometric indicator detected via the patient-controlled drug-requesting device; and a pain assessment performed after a prior delivery of the drug in response to a prior request for the drug. Clause 18. The method of Clause 16 or Clause 17, further comprising: providing the patient physiological data to a machine-learning model; and receiving a drug-request authorization indication from the machine-learning model, wherein the drug-request authorization indication is used to determine whether the patient is authorized to receive a requested dose of a drug.

Clause 19. The method of any one of Clauses 16 through 18, further comprising: prior to identifying the one or more drug-delivery apparatuses: forming an electronic communication connection with a lockbox control interface configured to enclose and prevent access to at least a portion of the one or more drug-delivery apparatuses; receiving a wireless credential at an input interface of the infusion control device; transmitting, by the infusion control device, the wireless credential to the one or more drug-delivery apparatuses via the electronic communication connection with the lockbox control interface; receiving an indication from the one or more drug-delivery apparatuses via the electronic communication connection, after the transmitting, whether the patient is authorized to request doses of the drug to be delivered; and in accordance with receiving an indication that the patient is authorized to request doses of the drug to be delivered, using the drug-control algorithm to determine if a drug request submitted by the patient is an authorized drug request.

Clause 20. The method of any one of Clauses 16 through 19, wherein: detecting the one or more drug-delivery apparatuses comprises: when a respective drug-delivery apparatus is a first type of drug-delivery apparatus including a syringe configured to be manually pressed to inject a first drug into the patient, selecting a first algorithm for delivering the drug via the first type of drug-delivery apparatus as the drug-control algorithm, the first algorithm including instructions for causing the syringe to inject the first drug into the patient; and when the respective drug-delivery apparatus is a second type of drug-delivery apparatus including an intravenous infusion pump that is integrally coupled with an electronic actuating system for causing doses of a second drug to be delivered to the patient, selecting a second algorithm for delivering the drug via the second type of drug-delivery apparatus as the drug-control algorithm, the second algorithm including instructions for causing delivery via the intravenous infusion pump.

Clause 21. The method of any one of Clauses 16 through 20, further comprising: determining one or more therapies that the patient-controlled drug-requesting device is able to receive drug requests for from the patient; based on determining one or more therapies enabled by the patient-controlled drug-requesting device, selecting one or more drug-control algorithms for each respective therapy of the one or more therapies; and providing each respective drug-control algorithm of the one or more drug-control algorithms for use in conjunction with the patient-controlled drug-requesting device.

Clause 22. The method of any one of Clauses 16 through 21, further comprising: after the drug-control algorithm is selected: detecting electronic connection of a different device to the infusion control device; identifying a second drug-control algorithm, associated with the different device and distinct from the drug-control algorithm, for authorizing requests for doses of the drug to the patient; and replacing a selected drug-control algorithm with the second drug-control algorithm. Clause 23. The method of any one of Clauses 16 through 22, wherein the drug-control algorithm is configured to prevent the drug delivery of a particular drug while the patient physiological data does not meet one or more delivery-control criteria that are required for authorizing doses of the drug to be delivered.

Clause 24. The method of Clause 23, further comprising: after preventing the drug delivery of the particular drug based on a determination that a patient is not authorized to perform the request: preventing the patient from receiving delivery of the drug for a predetermined amount of time. Clause 25. A non-transitory computer-readable storage medium comprising instructions which, when executed by one or more processors of an infusion control device in operable communication with one or more drug-delivery apparatuses, cause performance of operations, comprising: detecting a patient-controlled drug-requesting device that is in operable communication with the infusion control device; identifying, based on detecting the patient-controlled drug-requesting device, (i) one or more sensor devices, and (ii) one or more drug-delivery apparatuses separate from the infusion control device, wherein the one or more sensor devices and the one or more drug-delivery apparatuses are in operable communication with the infusion control device; selecting, based on identifying the one or more sensor devices and the one or more drug-delivery apparatuses, a drug-control algorithm for approving drug requests received by the patient-controlled drug-requesting device; identifying a patient using the patient-controlled drug-requesting device; receiving patient physiological data from the one or more sensor devices in operable communication with the infusion control device; receiving a request for a drug to be delivered to the patient by a drug-delivery apparatus of the one or more drug-delivery apparatuses; determining whether the patient is authorized to perform the drug request based on a function of the drug-control algorithm, the patient physiological data, and a history of drug administrations to the patient; and causing, based on determining, using the drug-control algorithm, that the patient is authorized to perform the drug request, delivery of the drug via the one or more drug-delivery apparatuses.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented. The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The term website, as used herein, may include any aspect of a website, including one or more web pages, one or more servers used to host or store web related content, etc. Accordingly, the term website may be used interchangeably with the terms "web page" and "server." The predicate words "configured to," "operable to," and "programmed to" do not imply any particular tangible or intangible modification of a subject, but, rather, are intended to be used interchangeably. For example, a processor configured to monitor and control an operation, or a component may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all implementations, or one or more implementations. An embodiment may provide one or more examples. A phrase such as an "embodiment" may refer to one or more implementations and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein, the terms "determine" or "determining" encompass a wide variety of actions. For example, "determining" may include calculating, computing, processing, deriving, generating, obtaining, looking up (e.g., looking up in a table, a database, or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine-readable aggregation of information such as an XML document, fixed field message, comma separated message, or the like. A message may, in some implementations, include a signal utilized to transmit one or more representations of the information. While recited in the singular, it will be understood that a message may be composed, transmitted, stored, received, etc. in multiple parts. As used herein a "user interface" (also referred to as an interactive user interface, a graphical user interface or a UI) may refer to a network-based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, web services, or rich site summary (RSS). In some implementations, a UI may be included in a stand-alone client (for example, thick client, fat client) configured to communicate (e.g., send or receive data) in accordance with one or more of the aspects described. The communication may be to or from a medical device, diagnostic device, monitoring device, or server in communication therewith.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more obj ects, data sets, information and/or the like so to appear to be the same or equal. Correspondence may be assessed using one or more of a threshold, a value range, fuzzy logic, pattern matching, a machine-learning assessment model, or combinations thereof.

## Claims

1. An infusion control device, comprising:
one or more processors; and
a memory storing instructions which, when executed by the one or more processors, cause the one or more processors to perform operations comprising:
detecting a patient-controlled drug-requesting device that is in operable communication with the infusion control device;
identifying (i) one or more sensor devices, and (ii) one or more drug-delivery apparatuses separate from the infusion control device, in operable communication with the infusion control device, wherein the one or more sensor devices and the one or more drug-delivery apparatuses are in operable communication with the infusion control device;
selecting, based on identifying the one or more sensor devices and the one or more drug-delivery apparatuses, a drug-control algorithm for approving drug requests received by the patient-controlled drug-requesting device and for controlling delivery of one or more drugs to be delivered by the one or more drug-delivery apparatuses;
identifying a patient using the patient-controlled drug-requesting device;
receiving patient physiological data from the identified one or more sensor devices in operable communication with the infusion control device;
receiving a request for a drug to be delivered to the patient by a drug-delivery apparatus of the one or more drug-delivery apparatuses;
determining whether the patient is authorized to perform the drug request based on a function of the drug-control algorithm, the patient physiological data, and a history of drug administrations to the patient; and
causing, based on determining, using the drug-control algorithm, that the patient is authorized to perform the drug request, delivery of the drug via the one or more drug-delivery apparatuses.

2. The infusion control device of Claim 1, wherein the determining whether the patient is authorized to perform the request is based on satisfying one or more delivery-control criteria related to one or more of:
a determination that a biological indicator of the patient is beyond a patient health threshold for administering the drug;
a pharmacokinetic property of the drug;
a biometric indicator detected via the patient-controlled drug-requesting device; and
a pain assessment performed after a prior delivery of the drug in response to a prior request for the drug.

3. The infusion control device of Claim 1, wherein the operations further comprise:
providing the patient physiological data to a machine-learning model; and
receiving a drug-request authorization indication from the machine-learning model, wherein the drug-request authorization indication is used to determine whether the patient is authorized to receive a requested dose of a drug.

4. The infusion control device of Claim 1, wherein the operations further comprise, prior to identifying the one or more drug-delivery apparatuses:
forming an electronic communication connection with a lockbox control interface configured to enclose and prevent access to at least a portion of the one or more drug-delivery apparatuses;
receiving a wireless credential at an input interface of the infusion control device;
transmitting, by the infusion control device, the wireless credential to the one or more drug-delivery apparatuses via the electronic communication connection with the lockbox control interface;
receiving an indication from the one or more drug-delivery apparatuses via the electronic communication connection, after the transmitting, whether the patient is authorized to request doses of the drug to be delivered; and
in accordance with receiving an indication that the patient is authorized to request doses of the drug to be delivered, using the drug-control algorithm to determine if a drug request submitted by the patient is an authorized drug request.

5. The infusion control device of Claim 1, wherein:
detecting the one or more drug-delivery apparatuses comprises:
when a respective drug-delivery apparatus is a first type of drug-delivery apparatus including a syringe configured to be manually pressed to inject a first drug into the patient, selecting a first algorithm for delivering the drug via the first type of drug-delivery apparatus as the drug-control algorithm, the first algorithm including instructions for causing the syringe to inject the first drug into the patient; and
when the respective drug-delivery apparatus is a second type of drug-delivery apparatus including an intravenous infusion pump that is integrally coupled with an electronic actuating system for causing doses of a second drug to be delivered to the patient, selecting a second algorithm for delivering the drug via the second type of drug-delivery apparatus as the drug-control algorithm, the second algorithm including instructions for causing delivery via the intravenous infusion pump.

6. The infusion control device of Claim 1, wherein the operations further comprise:
determining one or more therapies that the patient-controlled drug-requesting device is able to receive drug requests for from the patient;
based on determining the one or more therapies enabled by the patient-controlled drug-requesting device, selecting one or more drug-control algorithms for each respective therapy of the one or more therapies; and
providing each respective drug-control algorithm of the one or more drug-control algorithms for use in conjunction with the patient-controlled drug-requesting device.

7. The infusion control device of Claim 1, wherein the operations further comprise, after the drug-control algorithm is selected:
detecting electronic connection of a different device to the infusion control device;
identifying a second drug-control algorithm, associated with the different device and distinct from the drug-control algorithm, for authorizing requests for doses of the drug to the patient; and
replacing a selected drug-control algorithm with the second drug-control algorithm.

8. The infusion control device of Claim 1, wherein the drug-control algorithm is configured to prevent delivery of a particular drug while the patient physiological data does not meet one or more delivery-control criteria that are required to be satisfied for authorizing doses of the drug to be delivered.

9. The infusion control device of Claim 8, wherein the operations further comprise, after preventing the drug delivery of the particular drug based on a determination that a patient is not authorized to perform the request:
preventing the patient from receiving delivery of the particular drug for a predetermined amount of time.

10. The infusion control device of Claim 9, wherein the operations further comprise:
while the patient is prevented from receiving delivery of the particular drug for the predetermined amount of time:
receiving a second request for another drug, different from the particular drug, to be delivered to the patient by another drug-delivery apparatus of the one or more drug-delivery apparatuses; and
based on the patient physiological data that did not meet the one or more delivery-control criteria for authorizing the request, determining that the patient is authorized to perform the second request for the other drug; and
causing, based on determining that the patient is authorized to perform the second request, delivery of the other drug via the one or more drug-delivery apparatuses.

11. The infusion control device of Claim 9, further comprising:
after determining that the patient is not authorized to perform the request, determining that the patient physiological data that did not meet the delivery-control criteria would meet other delivery-control criteria for authorizing a request for delivery of another drug; and
based on determining that another request for delivery of the other drug would be authorized based on the patient physiological data, prompting the patient that the other drug is available to be delivered.

12. The infusion control device of Claim 1, wherein the identifying of the one or more sensor devices, and the one or more drug-delivery apparatuses is performed based on detecting that the patient-controlled drug-requesting device is in operable communication with the infusion control device.

13. The infusion control device of Claim 1, further comprising:
a body, the body comprising:
a display, and
one or more universal coupling receptacles configured to receive respective sub-assemblies of the infusion control device;
one or more sub-assemblies, wherein:
each of a plurality of sub-assemblies is configured to form an operable connection with each of the universal coupling receptacles of the body;
each of the one or more sub-assemblies comprise respective constituent electronic components of the infusion control device; and
each of the one or more sub-assemblies comprise universal connectors configured to operably couple with the one or more universal coupling receptacles of the body, wherein, in accordance with a respective universal connector of a respective sub-assembly of the one or more sub-assemblies becoming coupled with a respective connection point of the body, constituent electronic components of the infusion control device become operably coupled with other constituent electronic components of the body.

14. The infusion control device of Claim 13, wherein each of the one or more universal coupling receptacles is configured to be coupled with one of the following:
a patient care unit comprising proprietary software distinct from default software of the infusion control device;
a lockbox and/or a lockbox control interface associated with the lockbox, and/or a hardware shelf associated with the lockbox; and
a sensor device of the one or more sensor devices, distinct from another sensor device associated with the one or more sub-assemblies.

15. A method, comprising:
at an infusion control device that includes one or more processors and a memory storing instructions:
detecting a patient-controlled drug-requesting device that is in operable communication with the infusion control device;
identifying, based on detecting the patient-controlled drug-requesting device, (i) one or more sensor devices, and (ii) one or more drug-delivery apparatuses separate from the infusion control device, wherein the one or more sensor devices and the one or more drug-delivery apparatuses are in operable communication with the infusion control device;
selecting, based on identifying the one or more sensor devices and the one or more drug-delivery apparatuses, a drug-control algorithm for approving drug requests received by the patient-controlled drug-requesting device;
identifying a patient using the patient-controlled drug-requesting device;
receiving patient physiological data from the one or more sensor devices in operable communication with the infusion control device;
receiving a request for a drug to be delivered to the patient by a drug-delivery apparatus of the one or more drug-delivery apparatuses;
determining whether the patient is authorized to perform the drug request based on a function of the drug-control algorithm, the patient physiological data, and a history of drug administrations to the patient; and
causing, based on determining, using the drug-control algorithm, that the patient is authorized to perform the drug request, delivery of the drug via the one or more drug-delivery apparatuses.

16. The method of Claim 15, wherein the determining whether the patient is authorized to perform the request is based on satisfying one or more delivery-control criteria related to one or more of:
a determination that a biological indicator of the patient is beyond a patient health threshold for administering the drug;
a pharmacokinetic property of the drug being requested;
a biometric indicator detected via the patient-controlled drug-requesting device; and
a pain assessment performed after a prior delivery of the drug in response to a prior request for the drug.

17. The method of Claim 15, further comprising:
providing the patient physiological data to a machine-learning model; and
receiving a drug-request authorization indication from the machine-learning model, wherein the drug-request authorization indication is used to determine whether the patient is authorized to receive a requested dose of a drug.

18. The method of Claim 15, further comprising:
prior to identifying the one or more drug-delivery apparatuses:
forming an electronic communication connection with a lockbox control interface configured to enclose and prevent access to at least a portion of the one or more drug-delivery apparatuses;
receiving a wireless credential at an input interface of the infusion control device;
transmitting, by the infusion control device, the wireless credential to the one or more drug-delivery apparatuses via the electronic communication connection with the lockbox control interface;
receiving an indication from the one or more drug-delivery apparatuses via the electronic communication connection, after the transmitting, whether the patient is authorized to request doses of the drug to be delivered; and
in accordance with receiving an indication that the patient is authorized to request doses of the drug to be delivered, using the drug-control algorithm to determine if a drug request submitted by the patient is an authorized drug request.

19. The method of Claim 15, wherein:
detecting the one or more drug-delivery apparatuses comprises:
when a respective drug-delivery apparatus is a first type of drug-delivery apparatus including a syringe configured to be manually pressed to inject a first drug into the patient, selecting a first algorithm for delivering the drug via the first type of drug-delivery apparatus as the drug-control algorithm, the first algorithm including instructions for causing the syringe to inject the first drug into the patient; and
when the respective drug-delivery apparatus is a second type of drug-delivery apparatus including an intravenous infusion pump that is integrally coupled with an electronic actuating system for causing doses of a second drug to be delivered to the patient, selecting a second algorithm for delivering the drug via the second type of drug-delivery apparatus as the drug-control algorithm, the second algorithm including instructions for causing delivery via the intravenous infusion pump.

20. A non-transitory computer-readable storage medium comprising instructions which, when executed by one or more processors of an infusion control device in operable communication with one or more drug-delivery apparatuses, cause performance of operations, comprising:
detecting a patient-controlled drug-requesting device that is in operable communication with the infusion control device;
identifying, based on detecting the patient-controlled drug-requesting device, (i) one or more sensor devices, and (ii) one or more drug-delivery apparatuses separate from the infusion control device, wherein the one or more sensor devices and the one or more drug-delivery apparatuses are in operable communication with the infusion control device;
selecting, based on identifying the one or more sensor devices and the one or more drug-delivery apparatuses, a drug-control algorithm for approving drug requests received by the patient-controlled drug-requesting device;
identifying a patient using the patient-controlled drug-requesting device;
receiving patient physiological data from the one or more sensor devices in operable communication with the infusion control device;
receiving a request for a drug to be delivered to the patient by a drug-delivery apparatus of the one or more drug-delivery apparatuses;
determining whether the patient is authorized to perform the drug request based on a function of the drug-control algorithm, the patient physiological data, and a history of drug administrations to the patient; and
causing, based on determining, using the drug-control algorithm, that the patient is authorized to perform the drug request, delivery of the drug via the one or more drug-delivery apparatuses.
